# EUROPÄISCHE PATENTANMELDUNG

(11) **EP 4 778 909 A1**
(43) Veröffentlichungstag der Anmeldung: **22.07.2026**
(21) Anmeldenummer: 25152012.8
(22) Anmeldetag: 15.01.2025
(51) Int. Cl.: C07C 5/48, C07C 11/04, C07C 7/00, C07C 7/09, C01B 3/00, C10K 1/00

(54) **VERFAHREN UND ANLAGE ZUR OXIDATIVEN DEHYDRIERUNG VON ETHAN**

(71) Anmelder: Linde GmbH, 82049 Pullach (DE)
(72) Erfinder: Meiswinkel, Andreas, 83253 Rimsting (DE); Schubert, Martin, 81248 München (DE); Tota, Desislava, 81479 München (DE); Fritz, Helmut, 81375 München (DE); Zellhuber, Mathieu, 82152 Martinsried (DE); Wöhl, Anina, 30966 Hemmingen (DE); Zander, Hans-Jörg, 81479 München (DE); Dobmaier, Sonja, 82515 Wolfratshausen (DE)

(57) **Zusammenfassung**

Es wird ein Verfahren (100, 200, 300) zur oxidativen Dehydrierung von Ethan vorgeschlagen, wobei ein erstes Komponentengemisch unter Erhalt eines zweiten Komponentengemischs einem oder mehreren Umsetzungsschritten (10) unterworfen wird, wobei der eine oder die mehreren Umsetzungsschritte (10) die oxidative Dehydrierung von Ethan umfasst oder umfassen, wobei das zweite Komponentengemisch oder ein Teil hiervon zur Bildung eines dritten Komponentengemischs verwendet wird, wobei das dritte Komponentengemisch einer Rohgasbehandlung (50) unterworfen wird, und wobei in der Rohgasbehandlung (50) Sauerstoff und Ethin in dem dritten Komponentengemisch umgesetzt werden. Es ist vorgesehen, dass die Rohgasbehandlung (50) unter Verwendung eines oder mehrerer, Festbettreaktoren durchgeführt wird und dass ein Gehalt an Sauerstoff und Kohlenmonoxid in dem dritten Komponentengemisch eingestellt wird, indem ein oder oder mehrere rückgeführte vierte Komponentengemische und/oder Sauerstoff bei der Bildung des dritten Komponentengemischs verwendet wird oder werden. Eine entsprechende Anlage wird ebenfalls vorgeschlagen.

## Beschreibung

Die vorliegende Offenbarung betrifft ein Verfahren und eine Anlage zur oxidativen Dehydrierung von Ethan.

### Hintergrund

Die oxidative Dehydrierung (engl. Oxidative Dehydrogenation, ODH) von Alkanen mit zwei bis vier Kohlenstoffatomen ist grundsätzlich bekannt. Bei der oxidativen Dehydrierung werden die genannten Alkane mit Sauerstoff unter anderem zu den jeweiligen Alkenen und zu Wasser umgesetzt, wobei Nebenprodukte wie Kohlenmonoxid, Kohlendioxid, Alkine und höhere Kohlenwasserstoffe gebildet werden können und in einem Produktgemisch der oxidativen Dehydrierung ferner ein Rest nicht umgesetzter Alkane und Sauerstoff verbleiben kann. Typischerweise entsteht auch die jeweils mit dem eingesetzten Alkan korrespondierende (gesättigte und ggf. auch ungesättigte) Carbonsäure als Koppelprodukt. Insbesondere Alkine und Sauerstoff müssen aus dem Produktgemisch aus den unten erläuterten Gründen entfernt werden.

Die vorliegende Offenbarung betrifft die oxidative Dehydrierung von Ethan zu Ethen, die auch als ODHE bezeichnet wird, auch wenn an einigen Stellen allgemeiner auf die oxidative Dehydrierung ohne konkrete Nennung von Ethan als dem Edukt Bezug genommen werden sollte.

Es gilt, entsprechende Verfahren, insbesondere im Hinblick auf die Entfernung unerwünschter Nebenprodukte, zu verbessern

### Übersicht

Vor diesem Hintergrund werden ein Verfahren und eine Anlage zur oxidativen Dehydrierung von Ethan mit den Merkmalen der unabhängigen Patentansprüche vorgeschlagen. Ausgestaltungen sind Gegenstand der abhängigen Patentansprüche und der nachfolgenden Beschreibung.

Das vorgeschlagene Verfahren zur oxidativen Dehydrierung von Ethan umfasst, dass ein erstes Komponentengemisch unter Erhalt eines zweiten Komponentengemischs einem oder mehreren Umsetzungsschritten unterworfen wird, wobei der eine oder die mehreren Umsetzungsschritte in dem vorgeschlagenen Verfahren die oxidative Dehydrierung von Ethan umfasst oder umfassen.

In dem vorgeschlagenen Verfahren wird das zweite Komponentengemisch oder ein Teil hiervon zur Bildung eines dritten Komponentengemischs verwendet, wobei das dritte Komponentengemisch einer Rohgasbehandlung unterworfen wird. In der Rohgasbehandlung werden Sauerstoff und Ethin in dem dritten Komponentengemisch teilweise oder im Wesentlichen vollständig umgesetzt und hierdurch entfernt.

Das vorgeschlagene Verfahren sieht vor, dass die Rohgasbehandlung unter Verwendung eines oder mehrerer, insbesondere adiabat betriebener Festbettreaktoren durchgeführt wird, und dass ein Gehalt an Sauerstoff und/oder Kohlenmonoxid und/oder Ethin in dem dritten Komponentengemisch eingestellt wird, indem ein oder mehrere rückgeführte vierte Komponentengemische und/oder Sauerstoff bei der Bildung des dritten Komponentengemischs verwendet wird oder werden. Durch die Einstellung der Gehalte der genannten Komponenten ändert sich typischerweise auch deren Verhältnis zueinander, so dass sich sich für den Betrieb der Rohgasbehandlung vorteilhafte Verhältnisse von Sauerstoff zu Ethin und/oder Kohlenmonoxid zu Sauerstoff einstellen, wie weiter unten genauer angegeben.

Wie weiter unten im Detail ausgeführt, ermöglichen das vorgeschlagene Verfahren und seine Ausgestaltungen eine besonders vorteilhafte Beeinflussung der Reaktionsbedingungen in der Rohgasbehandlung, insbesondere einer Reaktionstemperatur und/oder eines Temperaturanstieges der Reaktionstemperatur, und hierüber weiter insbesondere eine Reduzierung eines Verlusts an Wertprodukten und einer Bildung von unerwünschten Nebenprodukten wie höheren Kohlenwasserstoffen. Das vorgeschlagene Verfahren und seine Ausgestaltungen umfassen eine Realisierung eines Regelungskonzeptes, bei dem eine Reihe von Restriktionen bekannter Verfahren überwunden werden, und das einen besonders effektiven und effizienten Betrieb einer Rohgasbehandlung ermöglicht. Es wird eine sehr weitreichende oder im Wesentlichen vollständige Entfernung unerwünschter Komponenten der erläuterten Art ermöglicht.

In dem vorgeschlagenen Verfahren und seinen Ausgestaltungen umfasst das erste Komponentengemisch insbesondere Dampf, das Ethan und Sauerstoff. Das zweite Komponentengemisch umfasst insbesondere einen Anteil des Ethans aus dem ersten Komponentengemisch, der in dem einen oder den mehreren Umsetzungsschritten nicht umgesetzt wurde, sowie Ethen, Ethin, Sauerstoff, sowie Kohlenmonoxid und Kohlendioxid. Das dritte Komponentengemisch umfasst zumindest einen Teil desEthans, Ethens und Ethins sowie des Sauerstoffs und des Kohlenmonoxids aus dem zweiten Komponentengemisch. Das zweite Komponentengemisch ist insbesondere ein Produktgemisch der oxidativen Dehydrierung, das auch noch die in der oxidativen Dehydrierung gebildeten Koppel- bzw. Nebenprodukte Wasser und Essigsäure umfasst. Die Bildung eines dritten Komponentengemischs unter Verwendung des zweiten Komponentengemischs oder eines Teils hiervon erfolgt in Form einer Kondensation von Wasser und Essigsäure bzw. umfasst diese. Das dritte Komponentengemisch ist daher gegenüber dem zweiten Komponentengemisch an Wasser und Essigsäure abgereichert bzw. im Wesentlichen frei hiervon. Damit umfasst der Begriff "abgereichert" auch eine Abreicherung auf Null.

Das zweite und dritte Komponentengemisch umfassen in dem vorgeschlagenen Verfahren insbespondere vorwiegend Ethen und Ethin als Produkt und Nebenprodukt, was aber das Vorhandensein höherer Alkane, Alkene und Alkine nicht ausschließt, da solche Verbindungen ebenfalls als Nebenprodukte gebildet werden können.

In dem vorgeschlagenen Verfahren und seinen Ausgestaltungen kann vorgesehen sein, dass ein oder mehrere Teilströme eines oder mehrerer, in der Rohgasbehandlung oder stromab hiervon gebildeter Stoffströme als das eine oder die mehreren vierten Komponentengemische verwendet wird oder werden. Ein "in der Rohgasbehandlung" gebildeter Stoffstrom kann dabei ein Stoffstrom sein, der von einer (insbesondere adiabat betriebenen) Reaktorstufe einer mehrstufigen Rohgasbehandlung in eine nachgeordnete (ebenfalls insbesondere adiabat betriebene) Reaktorstufe sein. Stromab der Rohgasbehandlung vorliegende, für den genannten Zweck infrage kommende Stoffströme können beliebige, aufbereitete, verdichtete, abgetrennte oder auf sonstige Weise gebildete Stoffströme sein. Die Auswahl solcher Stoffströme kann insbesondere auf Grundlage von Gehalten bestimmter Komponenten, beispielsweise von Kohlenmonoxid, erfolgen, wodurch beispielsweise eine Einstellung eines Gehalts an Sauerstoff und/oder Kohlenmonoxid bzw. ein Verhältnis dieser Komponenten in Bezug aufeinander oder auf das Ethin in dem dritten Komponentengemisch besonders vorteilhaft vorgenommen werden kann.

In dem vorgeschlagenen Verfahren und seinen Ausgestaltungen kann bzw. können das eine oder die mehreren vierten Komponentengemische in einer Gesamtmenge rückgeführt werden, die weniger als das 10-Fache, 5-Fache, 3-Fache oder 1-Fache der Menge eines nicht durch das eine oder die mehreren vierten Komponentengemische gebildeten Anteils des dritten Komponentengemischs entspricht. Die Verwendung derartiger, vergleichsweise geringer Rückführmengen ermöglicht es insbesondere, vorhandene Apparate ohne Erweiterung weiterzuverwenden bzw. die notwendigen Apparate entsprechend klein zu dimensionieren. Die geringen Rückführmengen können insbesondere dann verwendet werden, wenn entsprechende vierte Komponentengemische ausreichende Gehalte der jeweils für eine Einstellung des Sauerstoff- und/oder Kohlenmonoxidgehalts verwendbaren Komponenten aufweisen.

In dem vorgeschlagenen Verfahren und seinen Ausgestaltungen kann das vierte Komponentengemisch oder kann zumindest eines der mehreren vierten Komponentengemische gemeinsam mit, oder separat von, dem zweiten Komponentengemisch oder dem Teil hiervon, der zur Bildung des dritten Komponentengemischs verwendet wird, verdichtet werden. Da die Rohgasbehandlung vorteilhafterweise insbesondere auf einem höheren Druckniveau als stromabwärtige Schritte durchgeführt wird, kann eine entsprechende Verdichtung in einem solchen Fall vorgesehen sein. Je nach den Druckbedingungen stromauf der Rohgasbehandlung ist auch eine Verdichtung des zweiten Komponentengemischs oder dessen zur Bereitstellung des dritten Komponentengemischs verwendeten Teils erforderlich. In diesem Fall kann die erwähnte gemeinsame Verdichtung erfolgen. Falls nicht, kann auch die erwähnte separate Verdichtung im Rückführkreislauf durch einen sogenannten Kreislaufverdichter vorteilhaft sein.

In dem vorgeschlagenen Verfahren und seinen Ausgestaltungen kann bzw. können das vierte Komponentengemisch oder zumindest eines der mehreren vierten Komponentengemische unter Verwendung einer Leichtgasfraktion gebildet werden, die unter Verwendung eines stromab der Rohgasbehandlung durchgeführten Demethanisierungsschritts bereitgestellt wird. Eine derartige Leichtgasfraktion umfasst Methan und tiefer als Methan siedende Verbindungen, darunter insbesondere auch Kohlenmonoxid. Diese Leichtgasfraktion stellt daher eine Fraktion dar, die sich in besonderer Weise dafür eignet, den Kohlenmonoxidgehalt des dritten Komponentengemischs, und damit indirekt auch den Sauerstoffgehalt bzw. insbesondere das Verhältnis von Kohlenmonoxid zu Sauerstoff und die bereits erwähnten Verhältnisse, einzustellen. Andere Rückführströme können neben oder alternativ zu dieser Leichtgasfraktion ebenfalls verwendet werden. Dies sind insbesondere ein stromab der Rohgasbehandlung oder stromab eines der Rohgasbehandlung zugehörigen adiabaten Festbettreaktors abgezweigter Stoffstrom, ein stromab einer Kohlendioxidentfernung abgezweigter Stoffstrom, ein stromab eines Trockners abgezweigter Stoffstrom, ein stromab eines Deethanizers abgezweigter Stoffstrom, oder ein Ethanteilstrom eines Ethanrecycles eines C2-Splitters oder aus einem Seitenabzug eines C2-Splitters.

In dem vorgeschlagenen Verfahren und seinen Ausgestaltungen kann, alternativ oder zusätzlich zur erwähnten Rückführung des einen oder der mehreren vierten Komponentengemische, bei der Bildung des dritten Komponentengemischs Sauerstoff einstellbar, und an jeweils geeigneter Stelle, zudosiert werden. Dies ermöglicht eine besonders vorteilhafte und gezielte Beeinflussung des Sauerstoffgehalts, da typischerweise stromab der Rohgasbehandlung kein Sauerstoff mehr vorliegt und damit nicht über entsprechende Rückführströme bereitgestellt werden kann.

In dem vorgeschlagenen Verfahren und seinen Ausgestaltungen kann vorgesehen sein, dass eine oder mehrere Mengen des einen oder der mehreren, bei der Bildung des dritten Komponentengemischs verwendeten vierten Komponentengemischs und/oder des bei der Bildung des dritten Komponentengemischs verwendeten Sauerstoffs auf Grundlage einer Messung einer Konzentration von Sauerstoff, Kohlenmonoxid und/oder Ethin stromauf der Rohgasbehandlung und/oder auf Grundlage einer Messung eines adiabaten Temperaturanstieges in der Rohgasbehandlung eingestellt wird oder werden. Auf diese Weise können die in der Rohgasbehandlung vorliegenden Bedingungen vorteilhafterweise auf den jeweils gewünschten Werten bzw. in den gewünschten Wertebereichen gehalten werden.

In dem vorgeschlagenen Verfahren und seinen Ausgestaltungen kann vorgesehen sein, dass das vierte Komponentengemisch oder zumindest eines der mehreren vierten Komponentengemische, das dritte Komponentengemisch, und/oder zumindest ein weiteres Komponentengemisch stromab der Rohgasbehandlung unter Verwendung eines oder mehrerer Wärmetauscher temperiert wird oder werden. Auf diese Weise kann eine gezielte Temperaturanpassung erfolgen.

In dem vorgeschlagenen Verfahren und seinen Ausgestaltungen kann vorgesehen sein, dass der Gehalt an Sauerstoff und Kohlenmonoxid in dem dritten Komponentengemisch derart eingestellt wird, dass ein Volumenanteil von Sauerstoff mindestens dem 3-Fachen, mindestens dem 5-Fachen, mindestens dem 10-Fachen, mindestens dem 20-Fachen oder mindestens dem 25-Fachen eines Volumenanteils des Ethins entspricht. Ein Verhältnis von Kohlenmonoxid zu Sauerstoff (definiert als molares reaktionsstöchiometrisches Verhältnis eines Moleküls Kohlenmonoxid zu einem Atom Sauerstoff) kann bei mindestens 0,5, mindestens 0,7, mindestens 0,9, mindestens 1,1 oder mindestens 1,25 und höchstens 3,2 oder höchstens 3,0 liegen. Wird statt des molaren reaktionsstöchiometrischen Verhältnisses ein reguläres molekülbasiertes Verhältnis eines Kohlenmonoxidmoleküls zu einem Sauerstoffmolekül verwendet, ergeben sich jeweils die doppelten Werte.

In dem vorgeschlagenen Verfahren und seinen Ausgestaltungen kann ferner vorgesehen sein, dass das dritte Komponentengemisch mit einer Mindesttemperatur von von 160, 180, 200 oder 230 °C der Rohgasbehandlung zugeführt wird, wobei es sich hierbei um eine Temperatur bei Beginn eines Betriebs (Start-of-Run, SOR) handelt. Eine maximale Temperatur in der Rohgasbehandlung kann insbesondere bei 370, 360 oder 350 °C liegen und/oder die Rohgasbehandlung kann durch die vorgeschlagenen Maßnahmen derart betrieben werden, dass ein Temperaturanstieg in der Rohgasbehandlung um 40 bis 150, 60 bis 140 oder 70 bis 120 K erfolgt.

In dem vorgeschlagenen Verfahren und seinen Ausgestaltungen kann zudem vorgesehen sein, dass die Rohgasbehandlung auf einem Absolutdruck von 5 bis 50 bar, 15 bis 40 bar, 20 bis 35 bar oder 25 bis 35 bar und/oder mit einer stündlichen Gas-Raumgeschwindigkeit von 1.000 bis 15.000, 2.000 bis 12.000 oder 4.000 bis 10.000 h⁻¹ betrieben wird.

Schließlich kann in dem vorgeschlagenen Verfahren und seinen Ausgestaltungen vorgesehen sein, dass das dritte Komponentengemisch 25 bis 80 Vol.-%, 30-70 Vol.-% oder 40-60 Vol.% Ethen enthält und/oder die Rohgasbehandlung derart durchgeführt wird, dass ein maximaler Ethenverlust in der Rohgasbehandlung weniger als 2%, weniger als 1,5%, weniger als 1% oder weniger als 0,5% beträgt. Die Rohgasbehandlung kann insbesondere derart durchgeführt werden, dass ein Gehalt an Ethin Zielwert unter 1.000, 500 oder 250 vol.-ppp (Milliardstel Volumenanteile) Ethin, insbesondere 300 vol.-ppb Ethin, und ein Gehalt an Sauerstoff unter 100, 50, 20 oder 10 vol.-ppm (Millionstel Volumenanteile), insbesondere 10 vol.-ppm, beträgt.

Die genannten Gehalte an Sauerstoff und Kohlenmonoxid und die weiteren genannten Gehalte und Parameter ermöglichen eine Realisierung besonders vorteilhafter Bedingungen in der Rohgasbehandlung mit besonders geringen Verlusten an Wertprodukten und geringer Bildung von Nebenprodukten.

In dem vorgeschlagenen Verfahren und seinen Ausgestaltungen kann an einem Austritt der Rohgasbehandlung eine Sauerstoff- und/oder Ethinmessung durchgeführt und auf dieser Grundlage eine Menge des einen oder zumindest eines der mehreren rückgeführten vierten Komponentengemische eingestellt werden.

In dem vorgeschlagenen Verfahren und seinen Ausgestaltungen kann die Rohgasbehandlung unter Verwendung eines Katalysators durchgeführt werden, der mindestens eines der Elemente Kupfer, Mangan und Ruthenium aufweist. Entsprechende Katalysatoren sind besonders gut charakterisiert und ermöglichen eine vorteilhafte Beeinflussung in der erwähnten Weise.

Die vorgeschlagene Anlage zur oxidativen Dehydrierung von Ethan ist dafür eingerichtet, ein erstes Komponentengemisch unter Erhalt eines zweiten Komponentengemischs einem oder mehreren Umsetzungsschritten zu unterwerfen. Der eine oder die mehreren Umsetzungsschritte umfasst oder umfassen die oxidative Dehydrierung von Ethan. Die Anlage ist ferner dafür eingerichtet, das zweite Komponentengemisch oder einen Teil hiervon wird zur Bildung eines dritten Komponentengemischs zu verwenden, das dritte Komponentengemisch einer Rohgasbehandlung zu unterwerfen, und in der Rohgasbehandlung Sauerstoff und Ethin in dem dritten Komponentengemisch umzusetzen.

In der vorgeschlagenen Anlage sind für die Rohgasbehandlung ein oder mehrere, für einen adiabaten Betrieb eingerichtete Festbettreaktoren bereitgestellt, und es sind Mittel bereitgestellt, die dafür eingerichtet sind, einen Gehalt an Sauerstoff und Kohlenmonoxid im dritten Komponentengemisch einzustellen, indem ein oder mehrere rückgeführte vierte Komponentengemische und/oder Sauerstoff bei der Bildung des dritten Komponentengemischs verwendet wird oder werden.

Vorteile und Merkmale, die bezüglich des vorgeschlagenen Verfahrens und seinen Ausgestaltungen beschrieben wurden, gelten auch für die vorgeschlagene Anlage und umgekehrt. Diese werden entsprechend nur einmalig beschrieben, und auf die jeweiligen Erläuterungen kann verwiesen werden.

### Zeichnungen

Im Rahmen der vorliegenden Offenbarung vorgeschlagene Aspekte werden anhand der beiliegenden Zeichnung näher erläutert. Dabei zeigen
Figur 1 ein Verfahren zur oxidativen Dehydrierung von Ethan, das hier vorgeschlagenen Ausgestaltungen zugrunde liegen kann;
Figur 2 einen adiabaten Temperaturanstieg in Abhängigkeit von einem Sauerstoffgehalt am Eintritt einer Rohgasbehandlung;
Figur 3 Sauerstoff- und Ethinkonversionen in Abhängigkeit von einer Temperatur am Eintritt einer Rohgasbehandlung;
Figuren 4a und 4b Ethenverluste und eine Bildung von Nebenprodukten in Abhängigkeit von einer Temperatur am Eintritt einer Rohgasbehandlung;
Figur 5 eine Teilansicht einer vorgeschlagenen Ausgestaltung; und
Figur 6 eine Teilansicht einer vorgeschlagenen Ausgestaltung.

### Ausführungsformen

Die nachfolgend beschriebenen Ausführungsformen werden lediglich zu dem Zweck beschrieben, den Leser beim Verständnis der beanspruchten und zuvor erläuterten Merkmale zu unterstützen. Sie stellen lediglich repräsentative Beispiele dar und sollen hinsichtlich der Merkmale der Erfindung nicht abschließend und/oder beschränkend betrachtet werden. Es versteht sich, dass die zuvor und nachfolgend beschriebenen Vorteile, Ausführungsformen, Funktionen, Merkmale, Strukturen und/oder andere Aspekte nicht als Beschränkungen von Äquivalenten zu den Ansprüchen zu betrachten sind, und dass andere Ausführungsformen verwendet und Änderungen vorgenommen werden können, ohne vom Umfang der beanspruchten Erfindung abzuweichen.

Unterschiedliche Ausführungsformen der Erfindung können weitere zweckmäßige Kombinationen der beschriebenen Elemente, Komponenten, Merkmale, Teile, Schritte, Mittel usw. umfassen, aufweisen, aus ihnen bestehen oder im Wesentlichen aus diesen bestehen, auch wenn solche Kombinationen hier nicht spezifisch beschrieben sind. Darüber hinaus kann die Offenbarung andere Erfindungen umfassen, die gegenwärtig nicht beansprucht sind, die aber in Zukunft beansprucht werden können, insbesondere wenn sie vom Umfang der unabhängigen Ansprüche umfasst sind.

Erläuterungen, die sich auf Vorrichtungen, Apparate, Anordnungen, Systeme usw. gemäß Ausführungsformen der vorliegenden Erfindung beziehen, können auch für Verfahren, Prozesse, Methoden usw. gemäß den Ausführungsformen der vorliegenden Erfindung gelten und umgekehrt. Gleiche, gleich wirkende in ihrer Funktion einander entsprechende, baulich identische oder vergleichbar aufgebaute Elemente, Verfahrensschritte usw. können mit identischen Bezugszeichen angegeben sein.

Die nachfolgenden Erläuterungen und Definitionen, die einige Grundlagen der Erfindung betreffen, können für alle oder einen Teil der hier vorgestellten Ausgestaltungen gelten, und die Erläuterung bestimmter Aspekte im Zusammenhang mit nur einem Teil oder einer der Ausgestaltungen soll nicht dahingehend verstanden werden, dass diese Aspekte nicht auch mit anderen oder allen Ausgestaltungen, soweit technisch möglich und sinnvoll, verwirklicht sein können.

Sämtliche hier verwendete Prozentangaben können sich auf Mol-, Mengen- oder Volumenanteile beziehen. Druckangaben in bar sind, soweit nicht anders erläutert, insbesondere als Absolutdrücke zu verstehen.

Die Konjunktion "und/oder" soll, wenn in einer Aufzählung vor dem letzten Element der Aufzählung verwendet, so verstanden werden, dass alle in der Aufzählung genannten Begriffe in beliebiger Weise miteinander kombiniert werden können. Mit anderen Worten ist mit "A, B und/oder C" "A und/oder B und/oder C" oder "wenigstens eines der Elemente A, B, C in beliebiger Kombination" gemeint.

Ist vorliegend davon die Rede, dass beispielsweise ein Stoffstrom oder Komponentengemisch unter Verwendung eines anderen Stoffstroms oder Komponentengemischs, oder eines Teils des anderen Stoffstroms oder Komponentengemischs "gebildet" wird, können hierbei beliebige Schritte, wie beispielsweise Abzweigen von Teilmengen oder Teilströmen, Vereinigen mit anderen Komponentengemischen oder Stoffströmen, chemisches oder physikalisches Umsetzen zumindest einiger Komponenten, Erwärmen, Abkühlen, Verdampfen, Kondensieren, Verdichten, Entspannen usw. umfasst sein. Ein "Teil" eines Komponentengemischs oder Stoffstroms kann auf eine derartige Weise gebildet sein, und es versteht sich, dass ein "Bilden" eines Stoffstroms oder Komponentengemischs unter Verwendung eines anderen Stoffstroms oder Komponentengemischs die Verwendung eines nochmals anderen Stoffstroms oder Komponentengemischs oder einer Reinkomponente umfassen kann.

Die im Rahmen der vorliegenden Offenbarung verwendeten Begriffe haben grundsätzlich die in der Fachwelt anerkannten Bedeutungen. So sei zu den hier verwendeten Begriffen auf die eingangs zitierte Fachliteratur verwiesen.

Wie eingangs erwähnt, erstrecken sich hier vorgeschlagene Ausgestaltungen auf die oxidative Dehydrierung von Ethan. Die oxidative Dehydrierung von Ethan kann gegenüber etablierteren Verfahren zur Herstellung von Alkenen wie dem Steamcracken oder der katalytischen Dehydrierung vorteilhaft sein. So besteht aufgrund der Exothermie der beteiligten Reaktionen und der praktisch irreversiblen Wasserbildung keine thermodynamische Gleichgewichtslimitierung. Die oxidative Dehydrierung von Ethan kann bei vergleichsweise geringen Reaktionstemperaturen durchgeführt werden. Schließlich werden im Gegensatz zum Steamcracken geringere Mengen an wertlosen Nebenprodukten wie Koks gebildet.

Zu weiteren Details bezüglich der oxidativen Dehydrierung sei auf einschlägige Fachliteratur, beispielsweise Ivars, F. und López Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, sowie X. Li, E. Iglesia, Kinetics and Mechanism of Ethane Oxidation to Acetic Acid on Catalysts Based on Mo-V-Nb Oxides, J. Phys. Chem. C, 2008, 112, 15001-15008, verwiesen. Insbesondere haben sich für die oxidative Dehydrierung MoVNb- oder MoVTeNb- basierte Katalysatorsysteme als vielversprechend herausgestellt. Bei der oxidativen Dehydrierung werden insbesondere bei Verwendung solcher Katalysatoren unter industriell relevanten Reaktionsbedingungen signifikante Mengen der jeweiligen Carbonsäuren der eingesetzten Alkane als Nebenprodukte gebildet.

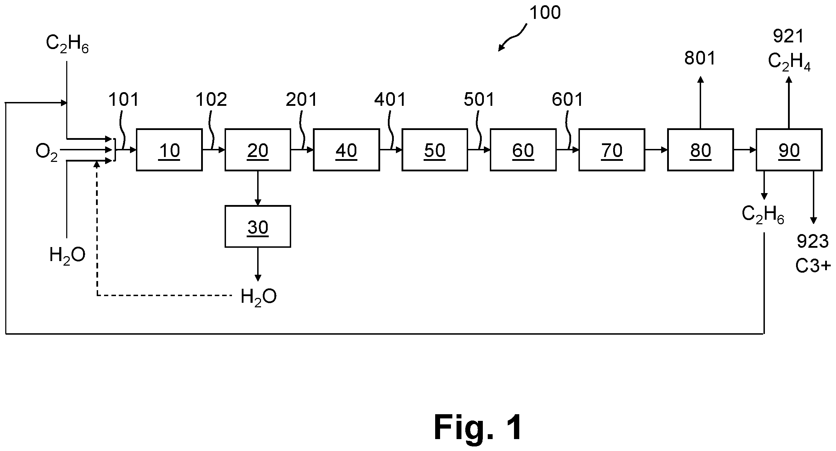

Figur 1 veranschaulicht ein Verfahren 100 zur oxidativen Dehydrierung von Ethan, das hier vorgeschlagenen Ausgestaltungen zugrunde liegen kann, welches aber auch selbst eine hier vorgeschlagene Ausgestaltung darstellen kann.

In dem Verfahren 100 werden Ethan C₂H₆, Sauerstoff O₂ und (insbesondere überhitzter) Wasserdampf H₂O als Einsatzstoffe bzw. Einsatzströme bereitgestellt und in geeigneter Weise zu einem Reaktionseintrittsstrom 101 vermischt, wie beispielsweise in EP 4 321 242 A1 offenbart, auf die hier und im Folgenden ausdrücklich Bezug genommen wird, und deren Inhalt, soweit zulässig, in vollem Umfang in die vorliegende Offenbarung aufgenommen ist. Der Reaktionseintrittsstrom stellt das zuvor und nachfolgend als "erstes Komponentengemisch" bezeichnete Komponentengemisch dar, und die Begriffe "Reaktionseintrittsstrom" und "erstes Komponentengemisch" werden synonym verwendet.

Insbesondere kann zur Bildung des ersten Komponentengemischs, d.h. des Reaktionseintrittsstroms 101, zunächst eine Vermischung von Ethan C₂H₆ und Dampf H₂O und erst danach die Einspeisung von Sauerstoff O₂ erfolgen. Das Ethan C₂H₆ kann vor der Mischung mit dem Dampf H₂O mittels Feed/Effluent-Wärmetauschern angewärmt werden. Der Sauerstoff O₂ wird typischerweise nicht angewärmt bzw. nur sehr geringfügig, d.h. insbesondere gerade derart, dass die Temperatur für Sauerstoffbrand nicht erreicht wird, aber ausreicht, dass eine Kondensation des Dampfs H₂O vermieden wird. Auf diese Weise kann eine Explosionsgefahr minimiert und unmittelbarer Kontakt von reinem Ethan C₂H₆ und Sauerstoff O₂ vermieden werden. Wie beispielsweise in WO 2022/194793 A1 beschrieben, kommt insbesondere reiner oder im Wesentlichen reiner Sauerstoff mit einem Gehalt von mindestens 95 vol.-% Sauerstoff, insbesondere mindestens 97 vol.-%, mindestens 99 vol.-% oder mindestens 99,5 vol.-% Sauerstoff, zum Einsatz. Der Sauerstoff kann insbesondere aus einer Luftzerlegungsanlage bereitgestellt werden. Aber auch andere Sauerstoffquellen wie beispielsweise aus einer Druckwechseladsorption, sowie die Verwendung von Luft oder in bestimmtem Umfang mit Sauerstoff angereicherter Luft, sind in entsprechenden Ausgestaltungen möglich.

Vor Eintritt in einen Umsetzungsschritt 10, der die oxidative Dehydrierung umfasst, erfolgt also, wie soeben erwähnt, bedarfsweise eine Anwärmung des ersten Komponentengemischs, d.h. des Reaktionseintrittsstroms 101, und/oder der Teilströme, aus denen dieses bzw. dieser gebildet wird, wobei dem Fachmann bekannte Wärmetauscher und insbesondere auch so genannte Feed-Effluent-Wärmetauscher sowie geeignete Regelungskonzepte verwendet werden können. Diese Anwärmung kann auch mehrstufig erfolgen und separate Anwärmungen der Einsatzstoffe oder entsprechender Mischungen vor und nach den jeweiligen Einspeisepunkten umfassen. Dabei gilt es eine Auskondensation von Wasser zu vermeiden. Insbesondere erfolgt in Ausgestaltungen die Einspeisung von (kaltem oder moderat erwärmtem) Sauerstoff möglichst kurz dem Umsetzungsschritt 10 oder einem entsprechenden Reaktor zu dessen Durchführung.

Der Umsetzungsschritt 10 kann beispielsweise unter Verwendung von MoVNbOx- oder MoVNbTeOx-Katalysatoren, insbesondere unter Verwendung eines Rohrbündelreaktors mit Salzschmelzekühlung, durchgeführt werden. Zur Einspeisung des Reaktionseintrittsstroms 101 können geeignete Einbauten und/oder Verteilsysteme, wie ebenfalls in z.B. in EP 4 321 242 A1 beschrieben, verwendet werden, um eine möglichst gleichmäßige Verteilung auf alle Rohre des Rohrbündelreaktors zu bewirken. Die Salzschmelze kann dabei im Gleich- oder Gegenstrom zur Flussrichtung des Reaktionseintrittsstroms 101 geführt werden, insbesondere im Gegenstrom, da hier die abgeführte Wärme aus den späteren Reaktionszonen in den vorderen Reaktionszonen genutzt werden kann. Als Kühlmittel kommen entsprechend dem jeweiligen Temperaturbereich der Reaktion Thermoöle oder insbesondere Salzschmelzen zur Anwendung. Zur Kühlung der Salzschmelze, d.h. zur Abfuhr der Reaktionswärme aus der Salzschmelze, kann insbesondere die Erzeugung von Dampf, insbesondere Satt-oder Hochdruckdampf, erfolgen, um der Aufwärmung durch die Reaktionswärme der oxidativen Dehydrierungsreaktion entgegenzusteuern.

Je nach Anlagenkapazität können auch mehrere Rohrbündelreaktoren parallel zur Durchführung des Umsetzungsschritts 10 eingesetzt werden. In der Eintrittszone eines Rohrbündelreaktors, in der die Reaktionsrohre üblicherweise mit einem Inertmaterial gefüllt sind, erfolgt die finale Anwärmung des Reaktionseintrittsstroms 101 auf die gewünschte Eintrittstemperatur in die eigentliche Reaktionszone. Diese nachfolgende Reaktionszone kann in Abschnitte unterteilt sein, die sich insbesondere in der Aktivität des verwendeten Katalysators unterscheiden können. Entsprechende Ausgestaltungen sind beispielsweise in WO 2019/243480 A1 beschrieben.

Die Produktverteilung kann dabei insbesondere durch Wahl der Prozessbedingungen, wie beispielsweise Verweilzeit, Lineargeschwindigkeit, Raumgeschwindigkeit, Eintrittsdruck und Partialdruck der Edukte beeinflusst werden. Insbesondere ermöglicht jedoch die Einstellung des Wasseranteils im Reaktionseintrittsstrom 101, insbesondere die Einstellung des Wasserpartialdrucks im heißen, d.h. vor der Kondensation, Reaktoraustrittsstrom, eine bedarfsgerechte Einstellung des Verhältnisses der Reaktionsprodukte Ethen und Essigsäure, wie beispielsweise in WO 2018/115416 A1 beschrieben. Dabei ist ein gewisser Mindestwasseranteil im Eintrittsstrom hilfreich oder notwendig, um eine stabile Katalysatoraktivität zu gewährleisten. Weiteres hierzu ist beispielsweise in WO 2018/115418 A1 ausgeführt. Katalysatoren und Prozessbedingungen sind beispielsweise in WO 2022/194793 A1 beschrieben.

Die oxidative Dehydrierung im Rohrbündelreaktor erfolgt im Fall der oxidativen Dehydrierung von Ethan typischerweise in einem Temperaturbereich zwischen 240 °C und 500°C, insbesondere zwischen 280 °C und 450 °C oder 300 °C und 400 °C, und bei einem Gesamtdruck des Reaktionseinsatzstromes 101 am Eintritt des Rohrbündelreaktors in einem Bereich zwischen 1 bar und 10 bar, insbesondere zwischen 2 bar und 6 bar Absolutdruck.

Typische Ethanumsätze im Umsetzungsschritt 10 reichen von 25% bis zu 75%, insbesondere 40% bis zu 60%. Das Verhältnis der Reaktionsprodukte Ethen und Essigsäure in einem Austrittsstrom 102 des Umsetzungsschritts 10 liegt in Abhängigkeit von den genauen Reaktions- und Prozessbedingungen in einem Bereich von 98:1 bis zu 70:30, insbesondere in einem Bereich von 95:2 bis zu 80:20 sowie in einem Bereich von 95:5 bis zu 90:10.

Während die oxidative Dehydrierung von Ethan typischerweise mit einem bestimmten Sauerstoffgehalt am Reaktoraustritt betrieben werden, sollte auf der anderen Seite der Sauerstoffgehalt am Reaktoraustritt, also im Austrittsstrom 102, und insbesondere vor dem Eintritt in einen nachfolgenden Zerlegungsteil, einen gewissen Grenzwert nicht überschreiten, um die folgenden Verfahrensschritte nicht durch zu hohe Sauerstoffkonzentrationen zu belasten, insbesondere auch um eine übermäßige Sauerstoffanreicherung und damit die mögliche Bildung einer explosionsfähigen Atmosphäre zu vermeiden.

Der Austrittsstrom 102 kann das zuvor und nachfolgend als "zweites Komponentengemisch" bezeichnete Komponentengemisch darstellen, und auch die Begriffe "Austrittsstrom" und "zweites Komponentengemisch" können hierin synonym verwendet werden.

Der Restgehalt an Sauerstoff im Austrittsstrom 102 kann bis zu wenigen Prozent betragen. Der Ethingehalt im Produktstrom 102 stromab des Umsetzungsschritts 10, also ebenfalls im noch feuchten, Essigsäure enthaltenden Prozessgas, liegt dagegen typischerweise unter einem Prozent. Die Gehalte an Kohlenmonoxid und Kohlendioxid belaufen sich unabhängig voneinander im Produktstrom 102 stromab des Umsetzungsschritts 10 auf wenige Prozent.

In dem erwähnten Kondensatabtrennschritt 20 erfolgt zunächst eine Kühlung des Austrittsstromes 102, also des zweiten Komponentengemischs. Hierbei erfolgt eine Kondensation eines Teiles des Austrittsstromes 102 und nachfolgend die Abscheidung und Abführung von Kondensat 202, das neben auskondensiertem Dampf und dem in dem Umsetzungsschritt 10 gebildeten Wasser insbesondere auch Essigsäure als Koppelprodukt der oxidativen Dehydrierung enthält. Nach der Abscheidung des Kondensats 202 verbleibt ein gasförmiger Strom als der erwähnte Folgestrom 201, der im Wesentlichen aus nicht umgesetztem Ethan, dem Reaktionsprodukt Ethen und den Nebenprodukten Kohlenmonoxid und Kohlendioxid besteht.

Der Folgestrom 201 wie auch ein später definerter Folgestrom 401 können das zuvor und nachfolgend als "drittes Komponentengemisch" bezeichnete Komponentengemisch darstellen, und auch die Begriffe "Folgestrom" und "drittes Komponentengemisch" können hierin synonym verwendet werden.

Auch Reste von Sauerstoff und weitere leichte Spurenkomponenten wie beispielsweise Ethin können in diesem Folgestrom 201 enthalten sein. Optional kann in dem Kondensatabtrennschritt 20 eine Waschkolonne, wie sie in WO 2018/115414 A1 beschrieben ist, zum Einsatz kommen. Weitere Ausgestaltungen können umfassen, dass ein erster Kondensatabtrennschritt lediglich unter Verwendung eines Abscheiders vor einer mehrstufigen Verdichtung 40 (siehe unten) erfolgt (Hauptabscheidung), welchem sich weitere Kondensatabtrennschritte nach jeder Verdichterstufe oder einem Teil der Verdichterstufen.

Insbesondere wenn die Essigsäure als eigenständiges Wertprodukt genutzt werden soll, wird das Kondensat 202 einer optionalen Essigsäureaufbereitung 30 zugeführt. Eine solche Essigsäureaufbereitung 30 nutzt dem Fachmann bekannte Verfahrensschritte, die insbesondere Extraktion und Rektifikation umfassen können und Essigsäure in gewünschter Reinheit, beispielsweise von mehr als 95%, mehr als 97% oder mehr als 99% gewinnen. Des Weiteren kann das in einer Essigsäureaufbereitung 30 gewonnene Wasser H₂O wiederum anteilig für die Erzeugung des zuvor erwähnten Wasserdampfes zur Bildung des Reaktionseintrittsstroms 101 genutzt werden, wie in Figur 1 entsprechend mit einem gestrichelten Pfeil veranschaulicht.

Der gasförmige Strom 201 wird der bereits erwähnten Verdichtung 40 zugeführt. Die Verdichtung 40 wird typischerweise in mehreren Stufen (üblicherweise drei oder vier bis sechs Stufen) durchgeführt und kann entsprechende Zwischenkühlungen und Kondensatabscheidungen zur Abtrennung von bei der Verdichtung 40 anfallenden Kondensatfraktionen, die hier als gemeinsame Kondensatfraktion 402 zusammengefasst werden, beinhalten. Ein in der Verdichtung 40 eingesetzter Prozessgaskompressor kann elektrisch mittels eines E-Motors oder mittels Dampf über (eine) entsprechende Dampfturbine(n) angetrieben werden. Aus der Verdichtung 40 wird ein Prozessstrom 401 auf einem erhöhten Druckniveau erhalten, dessen Zusammensetzung sich aus dem Eintrittsstrom 201 nach Entfernung der Kondensatfraktion 402 ergibt.

Der Prozessstrom 401 kann ebenfalls als das zuvor und nachfolgend als "drittes Komponentengemisch" bezeichnete Komponentengemisch verstanden werden, da sich seine Zusammensetzung typischerweise nicht, oder nur geringfügig im Wasser- und Essigsäuregehalt, von dem Folgestrom 201 unterscheidet.

Die Stoffströme 201 und 401, oder allgemein das dritte Komponentengemisch, enthalten neben Ethan, Ethen, Kohlenmonoxid und Kohlendioxid auch Sauerstoff entsprechend dem Sauerstoffrestgehalt im Austrittsstrom 102 des Umsetzungsschritts 10 in der oxidativen Dehydrierung, d.h. dem zweiten Komponentengemisch, sowie als Spurenkomponente gebildetes Ethin. Aufgrund der zuvor erfolgten Abtrennung der Kondensate 202 bzw. 402 sind Ethan, Ethen, Kohlenmonoxid, Kohlendioxid, Sauerstoff und Ethin in den Stoffströmen 201 bzw. 401 gegenüber dem Austrittsstrom 102 angereichert.

Im Hinblick auf eine mögliche weitere Anreicherung in nachgelagerten Verfahrensschritten eines Verfahrens 100 ist aus sicherheitstechnischen Überlegungen (genauere Ausführung dazu weiter unten) eine Sauerstoffentfernung aus dem Prozessgas erforderlich. Des Weiteren darf auch das Zielprodukt Ethen nur eine sehr geringe Sauerstoffkonzentration, typischerweise wenige vol.-ppb, aufweisen und erfordert somit eine strikte Sauerstoffentfernung. In gleicher Weise muss eine Ethinspezifikation im Ethenprodukt eingehalten werden. Diese liegt typischerweise bei unter 2 vol.-ppm (Millionstel Teile auf Volumenbasis), insbesondere unter 1 vol.-ppm. Es ist also im Rahmen eines Verfahrens 100 eine geeignete Ethinentfernung vorzusehen.

Hierzu sind gemäß Stand der Technik prinzipiell verschiedene Ansätze bekannt. Beispielsweise beschreibt US 8,519,210 B2 eine nachgeschaltete oder auch in einem Reaktor oder dem Umsetzungsschritt integrierte, katalytische Sauerstoffentfernung, wobei jedoch nur recht allgemein ein "oxygen elimination catalyst" spezifiziert wird. Auch nach WO 2017/144584 A1 wird ein Sauerstoffentfernungskatalysator bevorzugt im Reaktor stromab der Hauptreaktionszone eingesetzt. Beide Schriften gehen dabei nicht auf den Aspekt der Ethinentfernung ein.

Erst WO 2020/187572A1 sowie WO2018/153831A1 beschreiben einen Ansatz bei dem eine kombinierte Entfernung sowohl von Sauerstoff als auch von Ethin erfolgt, die vorliegend auch als Rohgasbehandlung 50 bezeichnet wird. Dabei wird anteilig jeweils auch Sauerstoff mit Kohlenmonoxid umgesetzt, wobei Kohlendioxid entsteht. Gemäß Stand der Technik, und wie in WO 2020/187572 A1 ausgeführt, erfolgt hier insbesondere die Verwendung von Katalysatoren, die mindestens eines der Elemente Kupfer, Mangan oder Ruthenium enthalten. Die Rohgasbehandlung 50 kann vor oder nach der Verdichtung 40 angeordnet sein. Besonders vorteilhaft ist, beispielsweise gemäß WO 2020/187572 A1 jedoch eine Anordnung der Rohgasbehandlung 50 von Sauerstoff und Ethin nach der Verdichtung 40 oder mindestens nach einer der Verdichtung 40 zugehörigen Verdichterstufe. Wie erwähnt, kann bei der Entfernung von Sauerstoff eine Reaktion mit Kohlenmonoxid und Ethin unter Bildung von Kohlendioxid und Wasser erfolgen, wodurch sich der Kohlendioxidgehalt im Prozessgasstrom entsprechend erhöht. Unabhängig von der genauen Anordnung und Reihenfolge von Verdichtung 40 und Rohgasbehandlung 50 wird also ein an Sauerstoff und Ethin abgereicherter bzw. armer Strom und ggf. an Kohlendioxid angereicherter Strom 501 gebildet.

Dieser Strom 501 wird einer Kohlendioxidentfernung 60 unterworfen, wobei ein an Kohlendioxid abgereicherter Folgestrom 601 gebildet wird. Die Entfernung von Kohlendioxid erfolgt herkömmlicherweise insbesondere mittels Absorption, beispielsweise regenerierbare Aminwäschen und nicht regenerierbare Laugewäschen. Kohlendioxid kann dabei aufgrund seiner hohen Wechselwirkung mit geeigneten Lösungsmitteln bzw. Waschflüssigkeiten vergleichsweise einfach aus dem Produktgemisch entfernt werden, wobei auch in hier vorgeschlagenen Ausgestaltungen bekannte Verfahren zur Kohlendioxidentfernung, insbesondere entsprechende Wäschen, beispielsweise Aminwäschen, zum Einsatz kommen können. Das beladene Waschmittel bei Aminwäschen wird dann in einer separaten Kolonne regeneriert, wobei im Wesentlichen reines Kohlendioxid durch Desorption freigesetzt wird und, wie nicht gesondert veranschaulicht, als relativ reiner Strom für eine weitere mögliche Verwendung zur Verfügung steht. Eine weitere Feinreinigung und Reduzierung des Kohlendioxidgehaltes ist durch eine (optionale, der Aminwäsche nachgeschaltete) Laugewäsche möglich.

Der an Kohlendioxid abgereicherte Folgestrom 601 wird sodann einer Trocknung 70 unterworfen, um in folgenden Verfahrensschritten die Bildung von Hydraten und/oder Eis zu verhindern. In der Trocknung 70 kommen dem Fachmann bekannte Trocknungsmittel, wie insbesondere Molekularsiebe oder Zeolithe, zum Einsatz. Auch die Verwendung spezieller Materialien, die eine Entfernung von Resten von Kohlendioxid oder auch von Spurenverbindungen wie Oxygenaten ermöglichen, ist grundsätzlich bekannt.

Der Trocknung 70 folgt ein sogenannter "kalter Teil", der insbesondere eine Demethanisierung 80 und eine Auf- und/oder Abtrennung 90 von Kohlenwasserstoffen mit zwei Kohlenstoffatomen voneinander und von drei Kohlenstoffatomen beinhalten kann. In der Demethanisierung 80 werden Kohlenmonoxid und andere leicht siedende Komponenten als Kopfstrom 801 abgetrennt. Die Auf- und/oder Abtrennung 90 umfasst insbesondere einen Splitter zur Trennung von Ethan und Ethen zur Gewinnung des Zielproduktes Ethen (Strom 921) in erforderlicher und spezifikationsgerechter Reinheit. Bedarfsweise kann auch ein Deethanizer vorgesehen sein, der einen Strom 923, der überwiegend Kohlenwasserstoffe mit drei Kohlenstoffatomen und schwerere Komponenten enthalten kann, als Sumpffraktion von einem Strom, der überwiegend die Kohlenwasserstoffe mit zwei Kohlenstoffatomen, d.h. Ethan und/oder Ethen, enthält. Die Anordnung von Splitter und Deethanizer kann dabei in geeigneter Weise erfolgen, wie grundsätzlich aus dem Stand der Technik bekannt.

Eine Anlage zur Durchführung eines des Verfahrens 100 beinhaltet ferner die notwendigen Hilfssysteme, wie z.B. insbesondere Dampf- und Kondensatsystem, Kesselspeisewassersystem, Regeneriersystem, Blowdown- und Fackelsystem, Kühlwassersystem, Propylen- und optional Ethen-Kältesystem, Stickstoffsystem, Druckluftsystem, Slop- und Abwassersystem, Ablaugebehandlung, Chemikalien- und Inhibitordosiersystem, Löschwassersystem sowie Tank- und Lagereinrichtungen für Edukte, Zwischenprodukte und Produkte.

Das in Figur 1 veranschaulichte Verfahren 100 und andere Verfahren umfassen, wie erläutert, eine Sauerstoff- bzw. Spurenentfernung, insbesondere von Ethin, wie erwähnt hier auch als Rohgasbehandlung 50 bezeichnet, im Prozessgas der oxidativen Dehdrierung. Wie zuvor dargelegt, ist für die odixative Dehydrierung ein Mindestsauerstoffanteil am Reaktoraustritt eines in dem Umsetzungsschritt 10 eingesetzten Reaktors sicherzustellen. Dieser Restsauerstoff sollte jedoch zumindest aus den nachfolgend erläuterten Gründen aus dem Prozessgasstrom 102 spezifikationsgerecht sowohl für folgende Prozessschritte des Verfahrens 100 als auch für Downstreamprozesse entfernt werden.

Ein ökonomischer Grund besteht in der Tatsache, dass in Gegenwart von Sauerstoff eine schnellere Alterung bzw. Zersetzung des in der Kohlendioxidwäsche 60 verwendeten Waschmittels (Amins) zu beobachten ist. Eine Sauerstoffentfernung in der Rohgasbehandlung stromauf der (regenerativen) Kohlendioxidentfernung 60 führt damit zu minimalem Verbrauch/Ersatz von Waschmittel.

Ein sicherheitstechnischer (und ökonomischer) Grund ist der, dass sich im Prozessgas 102 verbleibender Sauerstoff in der kryogenen Produktauftrennung, insbesondere im Kopfstrom des Demethanizers 80 stark aufkonzentriert, was zu kritischen und ggf. zündfähigen Gemischen aus Kohlenwasserstoff, Kohlenmonoxid und Sauerstoff im Kopfstrom des Demethanizers 80 führen kann. Folge wäre hier eine erforderliche Druckauslegung des Demethanizers 80 für sehr hohe Prozessdrücke, was entweder sehr hohe oder gar zu hohe Investitionskosten bedeutet oder für einen derartigen Druckbereich an die Baubarkeitsgrenzen stößt.

Eine Ethinentfernung aus dem Prozessgas ist aus Gründen der Produktreinheit des Ethens notwendig. Ethin wirkt insbesondere als Katalysatorgift in Downstreamprozessen, in denen das Produkt weiterverarbeitet wird.

Besonders relevant in entsprechenden Verfahren 100 sind also Aspekte zum Sauerstoff und Ethingehalt im Produktstrom 102 einer oxidativen Dehydrierung, insbesondere von Ethan, wie zuvor ausgeführt. Geeignete Katalysatoren und Anordnungen der Rohgasbehandlung 50 sind anderenorts beschrieben. Beispiele werden nachfolgend kurz erläutert.

WO 2020/187572 A1 sieht dabei insbesondere die Einspeisung von Wasserstoff für eine Rohgasbehandlung 50 als wesentliches Element vor. Eine andere Ausgestaltung einer Rohgasbehandlung 50 ist in WO 2018/153831 A1 beschrieben.

WO 2014/134703 A1 betrachtet spezifisch einen chemischen Komplex mit einem Ethancracker und Splitter, der durch eine oxidative Dehydrierung von Ethan ergänzt wird. Ähnlich wie US 8,519,210 B2 offenbart diese Schrift in diesem Zusammenhang unter anderem eine Sauerstoffentfernung als sogenannten Nachbrenner unmittelbar stromab eines Reaktors zur oxidativen Dehydrierung von Ethan. Diese Sauerstoffentfernung soll typischerweise bei Temperaturen deutlich unterhalb der Temperatur der eigentlichen Reaktion der oxidativen Dehydrierung von Ethan erfolgen. Als Katalysatoren werden Mischungen aus Mn₂O₃ und CuMn₂O₄ oder andere unter Bezug auf US 6,747,066 A und US 6,992,112 A als Referenz verwendet. Dieser Schritt wird explizit als "Oxygen Scavenging" beschrieben, insbesondere unter Verwendung eines Niedrigtemperaturreaktors im Bereich von 100 °C bis 400 °C. Hier werden Methan, Wasserstoff bzw. Kohlenmonoxid oxidativ umgesetzt. Diese Verbindungen können bedarfsweise dem Prozessstrom 102 auch zusätzlich beigemischt werden. Dies ist dem Fachmann bekannt und typisch für die aufgeführten Katalysatoren. Es werden typische Restsauerstoffgehalte von weniger als 1000 vol.-ppm nach diesem Verfahrensschritt genannt.

Eine sehr ähnliche Ausführung, nur teils unter Verwendung anderer Katalysatoren, wird auch in der US 2022/380277 A1 offenbart. Die US 11,447,434 B2 beschreibt unter anderem eine separate Oxidation von Kohlenmonoxid zur Sauerstoffentfernung sowie insbesondere eine separate Ethinentfernung unter Verwendung eines Katalysators, der ein Metall der Gruppe 11 des Periodensystems und einen Promoter (CeO₂ und ZrO₂) auf einem SiO₂-Träger verwendet.

In der US 8,519,210 B2 wird eine nachgeschaltete oder auch in einem Reaktor zur oxidativen Dehydrierung von Ethan integrierte, katalytische Sauerstoffentfernung beschrieben, wobei jedoch nur recht allgemein ein Sauerstoffeliminationskatalysator erwähnt wird. Dieser kann insbesondere Oxidationskatalysatoren, Verbrennungskatalysatoren und Hydrierkatalysatoren umfassen. Im beschreibenden Teil wird hier zur Sauerstoffentfernung eine Verbrennung vorzugsweise von Kohlenmonoxid und ggf. Kohlenwasserstoffen mit zwei und weniger Kohlenstoffatomen dargelegt, was einen entsprechenden Ausbeuteverlust bedeuten kann. Gemäß dieser Schrift kann also insbesondere ein vom eigentlichen Katalysator zur oxidativen Dehydrierung von Ethan unabhängiges und unterschiedliches Material zum Einsatz kommen und die zugrundeliegende Reaktion ist eine Umsetzung zu Kohlenmonoxid und/oder Kohlendioxid und Wasser.

Alle Dokumente zeigen jedoch keine Lösung für ein besonders vorteilhaftes Regelungs- und Betriebskonzept einer Sauerstoff- bzw. Spurenentfernung 50 im Kontext eines Gesamtverfahrens wie des beschriebenen Verfahrens 100 auf, sondern beschränken sich im Wesentlichen auf die Position innerhalb eines Gesamtverfahrens bzw. die Auswahl geeigneter Katalysatoren und allgemeiner Betriebsbedingungen.

Der Umsetzungsschritt 10 wird normalerweise so betrieben, dass die notwendige Mindestsauerstoffkonzentration sicher eingehalten wird. Dabei ist es nach Stand der Technik das Ziel die Mindestsauerstoffkonzentration gerade so weit zu überschreiten, dass ein in Bezug auf eine Katalysatorschädigung sicherer und stabiler Betrieb auch unter Berücksichtigung von möglichen, typischerweise aber nur geringfügigen, betriebstechnischen Schwankungen gewährleistet wird. Es wird also insbesondere die Sauerstoffaustrittskonzentration im Austrittstrom 102 des Umsetzungsschritts 10 bzw. im Folgestrom 201 nach der Essigsäureabtrennung 20 als betriebs- und sicherheitstechnisch wesentlicher Parameter zu Grunde gezogen und definiert somit i.d.R. die Zusammensetzung des Eintrittsstromes der Rohgasbehandlung 50.

Eine flexible Anpassung des Betriebes des Reaktors bzw. des Umsetzungsschritts auf die Anforderungen der Rohgasbehandlung 50 ist somit üblicherweise nicht vorgesehen oder technisch bzw. wirtschaftlich nicht sinnvoll möglich. Dennoch kann es aber im Rahmen des Betriebes eines Gesamtverfahrens 100 aber zu kurzzeitigen oder länger andauernden signifikanten Abweichungen der Sauerstoffkonzentration nach oben oder unten kommen, z.B. durch (unvorhergesehene) Betriebsstörungen oder Abweichungen vom Normalbetrieb des Reaktors, aber auch durch Anpassungen während des laufenden Betriebes. Auch beim Anfahren einer Anlage zur Durchführung des Verfahrens 100 ist es das Ziel, möglichst schnell einen stabilen Betrieb der Gesamtanlage zu erreichen und spezifikationsgerechte Produkte herzustellen (hier Ethen in Bezug auf den Anteil an Sauerstoff und Ethin). Diese Anforderungen machen jeweils eine schnelle und zuverlässige Anpassung des Betriebes der Rohgasbehandlung 50 erforderlich.

Dabei ist zu gewährleisten, dass in der Rohgasbehandlung 50, wie beispielsweise in Bezug auf das Verfahren 100 erläutert, die nachfolgend angegebenen Aufgaben gelöst bzw. Rahmenbedingungen eingehalten werden.

Sauerstoff und Ethin sind so weit zu entfernen, dass in nachfolgenden Prozessschritten eine Sauerstoffanreicherung sicher verhindert wird (Verhinderung der Bildung von zünd- bzw. explosionsfähigen Gemischen) und erforderliche Spezifikationen in Wertprodukten (hier insbesondere Ethen, z.B. insbesondere "PE-grade") zuverlässig eingehalten werden.

Bei den Reaktionen in der Rohgasbehandlung 50 handelt es sich um oxidative und stark exotherme Reaktionen. Entsprechende Wärmetönung durch katalytische Umsetzung begünstigt unselektive Reaktionen, die zu Verlust von Ethen und Ethan führen können. Gleichzeitig ist eine optimale Temperatur in einer Rohgasbehandlung 50 einzustellen, insbesondere am Reaktoreintritt und bei Verwendung eines dem Grunde nach bekannten adiabaten Festbettreaktors. Es gilt ein thermisches Durchgehen der Rohgasbehandlung zu vermeiden. Im vorliegenden Fall ist aufgrund der begrenzten Sauerstoffkonzentration im Eintrittsstrom der Rohgasbehandlung 50 zwar ein vollständiges klassisches Durchgehen zunächst oftmals selbstbegrenzend, aber es kann unter vollständigem Sauerstoffumsatz eine unerwünscht starke Ausprägung des Reaktionsgeschehens dicht am Reaktoreintritt erfolgen, was wiederum in diesem Bereich zu den zuvor genannten unselektiven Reaktionen sowie auch Katalysatorschädigungen durch Temperaturspitzen führen kann. Hier wirkt sich der Verlust von Ethen und die Bildung von Kohlenmonoxid und Kohlendioxid zusätzlich negativ aus.

Gemäß Stand der Technik muss auch aus diesen Gründen die oxidative Dehydrierung von Ethan so betrieben werden, dass die Sauerstoffkonzentration am Eintritt einer Rohgasbehandlung 50 einerseits - soweit vor dem beschriebenen Hintergrund technisch möglich - bereits möglichst minimiert und andererseits dann auch möglichst konstant gehalten wird. Insbesondere Abweichungen im Sauerstoffgehalt nach oben sind zu vermeiden, die wiederum die zuvor beschriebenen Effekte begünstigen und dann im Extremfall, also bei zu hoher Sauerstoffkonzentration, sogar zu einem thermischen Durchgehen führen können. Aber auch Abweichungen der Sauerstoffkonzentration nach unten können zu einer Verkokung oder Veränderung der katalytisch aktiven Spezies und somit reduzierten Leistungsfähigkeit und/oder Verkürzung der Standzeit des Katalysators in der Rohgasbehandlung 50 führen, die ggf. eine vorzeitige Regenerierung oder aber auch einen Austausch des Katalysators erfordert. Üblicherweise wird diesen Szenarien mit einer Abschaltung der Rohgasbehandlung 50 begegnet, was wiederum zu einem Produktionsausfall der Gesamtanlage zur oxidativen Dehydrierung von Ethan führt.

Gemäß Stand der Technik für stark exotherme Reaktionen können grundsätzlich für die Ausführung einer Rohgasbehandlung 50 geeignete Festbettreaktoren zum Einsatz kommen. Ein gekühlter Rohrbündelreaktor kann zwar hierbei zur effektiven Wärmeabfuhr und Temperaturkontrolle genutzt werden, stellt jedoch erheblichen apparativen Aufwand für den Reaktor an sich sowie für den erforderlichen Kühlkreislauf und die zugehörige Regelungstechnik dar. Hinzu kommt der vergleichsweise hohe Aufwand beim Befüllen und Entleeren des Katalysators.

Grundsätzlich sind aber auch adiabate Festbettreaktoren bekannt und geeignet. Insbesondere für diesen Reaktortyp gelten aber wiederum gewisse, nachfolgend erläuterte Rahmenbedingungen und Einschränkungen.

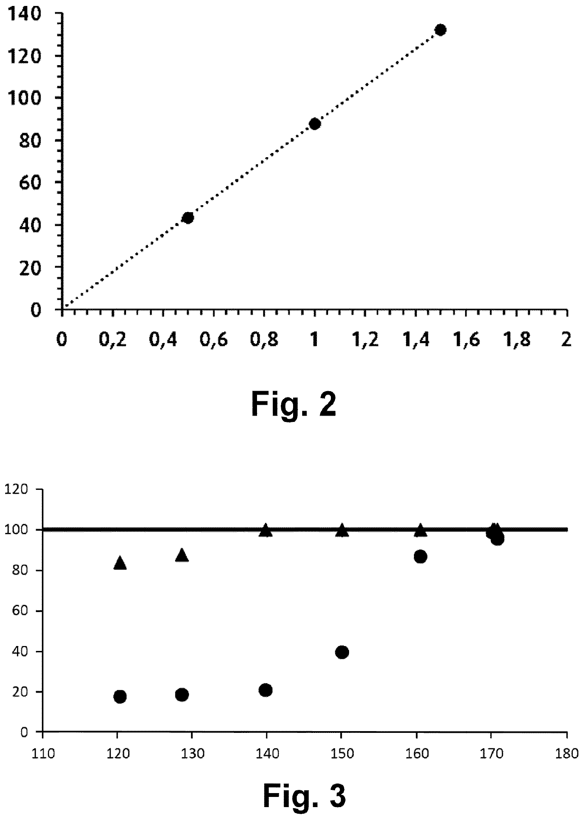

Die stark exotherme Reaktion führt über den Reaktor zu einem ausgeprägten Temperaturprofil bzw. im Falle eines adiabat betriebenen Festbettreakors insbesondere zu einem (sehr) hohen adiabaten Temperaturanstieg. Dies ist beispielhaft in Figur 2 für typisches Produktgas einer oxidativen Dehydrierung von Ethan, bestehend aus Ethan, Ethen, Kohlenmonoxid, Kohlendioxid und Ethin veranschaulicht. In Figur 1 ist dabei eine Sauerstoffeintrittskonzentration in mol% auf der Horizontalachse gegenüber einem adiabaten Temperaturanstieg in K auf der Vertikalachse verahschaulicht. Die Berechnung des zu erwartenden adiabaten Temperaturanstiegs ist möglich aus einer Betrachtung der vollständigen Umsetzung des Restsauerstoffs und des Ethins gemäß den nachfolgend angegebenen Reaktionsgleichungen (1) und (2).

C₂H₂ + 2,5 O₂ → 2 CO₂ + H₂O (1)

CO + 0,5 O₂ →CO₂ (2)

Eine Wärmeabführung kann im Wesentlichen erst in nachgelagerten Wärmetauschern erfolgen. Gleichzeitig ist eine Überreaktion oder ein Durchgehen des Reaktors zu verhindern. Eine Minimierung des Temperaturprofils über den Reaktor kann durch die Verwendung eines Verdünnungsmediums erfolgen, dies führt üblicherweise jedoch zu einer Vergrößerung des Reaktorvolumens, da ein größerer Volumen- bzw. Massenstrom durchgesetzt werden muss.

Üblicherweise kann der Austrittsstrom der Rohgasbehandlung 50 als Verdünnungsmedium genutzt werden. Um einen solchen Recycle zu erreichen ist jedoch zusätzlicher apparativer Aufwand (Rückverdichtung auf das Eintrittsdruckniveau) notwendig, insbesondere bei höheren Recycleraten. Gleichzeitig besteht die Gefahr der unerwünschten Umsetzung von Wertprodukten (im vorliegenden Fall z.B. die Oxidation von Ethen) durch den Recyclebetrieb. In der Praxis werden solche Recycleströme üblicherweise mit einer sehr hohen Recyclerate (zur Definition siehe unten) ausgeführt und betrieben. Zudem ist üblicherweise ein Mindestwert für die Recylerate vorgegeben, um einen sicheren und kontrollierten Betrieb zu gewährleisten. Insbesondere ein Teillastbetrieb oder die Anpassung an Betriebsschwankungen erfordert die Auslegung für einen weiten Betriebsbereich mit resultierenden zusätzlichen Anforderungen an das Equipment und die Regelungstechnik.

In der Praxis stellen aber auch die Anforderungen des Umsetzungsschritts 10, d.h. der oxidativen Dehydrierung, und einer Rohgasbehandlung 50 teils gegenläufige Anforderungen, wie nachfolgend erläutert.

Grundsätzlich kann der Umsetzungsschritt 10 zwar so betrieben werden, dass ein vorteilhaftes Verhältnis von Sauerstoff zu Ethin sowie von Kohlenmonoxid zu Sauerstoff am Eintritt der Rohgasbehandlung 50 eingehalten wird.

Eine oxidative Dehydrierung ist jedoch einerseits leichter betreib- und kontrollierbar, wenn eine höhere Sauerstoffkonzentration am Austritt des Umsetzungsschritts 10, d.h. in dem Stoffstrom 102 oder dem zweiten Komponentengemisch, gefahren wird. Dies wirkt sich unter anderem positiv auf den Katalysator zur oxidativen Dehydrierung und dessen Standzeit aus und vereinfacht Betriebsanpassungen. Zudem ist eine optimale Anpassung der Selektivität zu den Wertprodukten Ethen und Essigsäure bei höherer Austrittskonzentration von Sauerstoff möglich, bzw. dann, wenn die Austrittskonzentration von Sauerstoff als Freiheitsgrad verbleibt. Gleichzeitig können Fehlverteilungen bzw. Fehlverteilungen in der Katalysatorschüttung der Einzelrohre innerhalb eines Rohrbündelreaktors mit mehreren 10.000 Rohren toleriert werden.

Andererseits ist es für die Selektivität in der Rohgasbehandlung 50 jedoch vorteilhaft, das Verhältnis von Kohlenmonoxid zu Sauerstoff möglichst hochzuhalten, um Ethenverluste in der Rohgasbehandlung 50 zu minimieren. Hieraus resultiert also gemäß dem Stand der Technik eine obere Grenze für den Sauerstoffgehalt am Austritt des Reaktors zur oxidativen Dehydrierung bzw. des Umsetzungsschritts 10 in Bezug auf einen vorteilhaften und möglichst effizienten Betrieb der Gesamtanlage.

Gleichzeitig existiert bezüglich des Sauerstoffgehalts am Eintritt der Rohgasbehandlung 50 auch eine Untergrenze bzw. ein Mindestsauerstoffgehalt für deren spezifikationsgerechten Betrieb, d.h. insbesondere der spezifikationsgerechten Entfernung des Ethins.

Ausgehend von einer Mindesteintrittskonzentration für Sauerstoff am Eintritt der Rohgasbehandlung 50 beträgt der zu erwartende adiabate Temperaturanstieg mindestens ca. 40 K (für eine Sauerstoffeintrittskonzentration von 0,47 mol%, siehe Figur 2), wenn die Rohgasbehandlung über die oben angegebenen Reaktionen (Oxidation/Verbrennung des Ethins mit Sauerstoff und Umsetzung des restlichen Sauerstoffs mit Kohlenmonoxid, d.h. insbesondere ohne Ethenverlust) erfolgt. Ein entsprechend der Eintrittskonzentration für Sauerstoff (vgl. Figur 2) deutlich zu niedrigerer adiabater Temperaturanstieg deutet daher auf eine Minder- oder Fehlperformance der Rohgasbehandlung (unzureichende Sauerstoff- und /oder Ethinentfernung) hin. Ein entsprechend der Eintrittskonzentration von Sauerstoff deutlich zu hoher adiabater Temperaturanstieg deutet auf einen zu hohen Ethenverlust durch Verbrennung gemäß Reaktionsgleichung 3 hin. Diese Reaktion ist deutlich exothermer als die Reaktion gemäß obiger Reaktionsgleichung 2.

C₂H₄ + 3 O₂ → 2 CO₂ + 2 H₂O (3)

Wie in Figur 3 gezeigt, kann eine vollständige Entfernung von Sauerstoff und Ethin bei Temperaturen ab ca. 170 °C am Reaktoreintritt einer Rohgasbehandlung 50 erreicht werden. Figur 3 zeigt dabei eine Einlasstemperatur in °C auf der Horizontalachse gegenüber einer Konversionsrate in mol% auf der Vertikalachse. Werte für eine Sasuerstoffkonversion sind dabei in Form von Kreisen, Werte für eine Ethinkonversion in Form von Dreiecken veranschaulicht. Eine horizontale Linie bei 100% bezeichnet jeweils eine maximal erreichbare Gesamtkonversion von Sauerstoff bzw. Ethin. Die in Figur 2 veranschaulichten Ergebnisse wurden unter Verwendung eines CuO/MnObasierten Katalysators unter oxidativen Bedingungen erhalten. Die Reaktionsbedingungen umfassten einen Reaktionsdruck von 21 bara, eine stündliche Gas-Raumgeschwindigkeit von 3.700 h⁻¹, eine Einlasstemperatur, wie veranschaulicht, von 120 bis 170 °C, und einen Sauerstoffgehalt im Einsatz von 0,47 Vol%.

Die spezifikationsgerechte Entfernung nur von Ethin kann dabei bereits früher bei ca. 140 °C erreicht werden. Entsprechend Figur 2 kommt es in einem adiabaten Reaktor für die Rohgasbehandlung 50 zu einem Temperaturanstieg, der insbesondere von der Sauerstoffeintrittskonzentration abhängig ist. Beide Größen, die Reaktoreintrittstemperatur und der adiabate Temperatureinstieg, besitzen also eine Auswirkung auf den Entfernungsgrad von Sauerstoff und Ethin.

Obwohl gemäß Figur 3 eine nahezu vollständige Entfernung von Ethin und Sauerstoff bereits bei ca. 170 °C erreicht werden kann, zeigte sich, dass der Katalysator bei dieser Temperatur noch keine stabile Performance aufweist. Dies betrifft insbesondere die Sauerstoffentfernung, was einer relativ raschen Katalysatordeaktivierung zugeschrieben werden kann. Eine stabile Langzeitperformance wurde bei Katalysatorbetteintrittstemperaturen von über 200 °C, insbesondere von mind. 230 °C erreicht. Diese Werte gelten insbesondere für Start-of-Run-(SOR-)Bedingungen bzw. unmittelbar nach Regenerierung des Katalysators und können sich im Betrieb mit der Laufzeit dann nach oben verschieben.

Die Mindesttemperatur für einen stabilen Betrieb beträgt somit mindestens 200 °C, besonders bevorzugt mindestens 230 °C. Eine zu hohe Katalysatorbetttemperatur bzw. eine zu hohe Austrittstemperatur für einen adiabaten Reaktor führt zu deutlich erhöhten Ethenverlusten einerseits und Bildung von unerwünschten Nebenprodukten (hier insbesondere höheren aliphatischen und nicht aliphatischen Kohlenwasserstoffen), wodurch die Wirtschaftlichkeit des Gesamtprozesses negativ beeinflusst wird. Als maximal zulässige Temperatur hat sich in diesem Zusammenhang ca. 400 °C, insbesondere ca. 370 °C erwiesen.

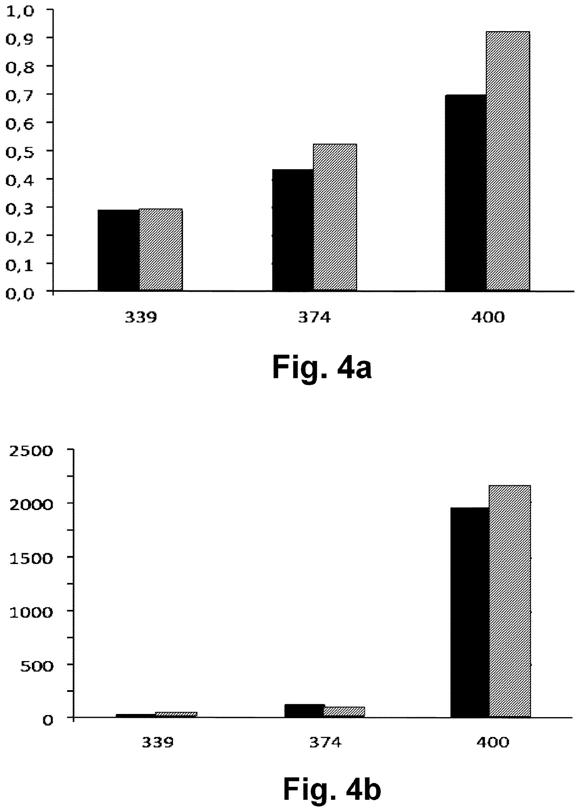

In Figur 4a und 4b sind jeweils Maximaltemperaturen in °C auf der Horizontalachse gegenüber einem Ethenverlust in% auf der Vertikalachse (Figur 4a) bzw. einer Bildung von Nebenprodukten in Form von Kohlenwasserstoffen mit drei bis sechs Kohlenstoffatomen in Gew-ppm veranschaulicht. Die jeweils für die Maximaltemperaturen von 339, 374 und 400 °C links dargestellten Balken zeigen Ergebnisse, die bei konstanter Sauerstoffkonzentration von 1,5 Vol% durch eine Variation der Reaktionseintrittstemperatur (230, 270 und 300 °C) erhalten wurden, die rechts dargestellten Balken dagegen Ergebnisse, die durch Variation der Sauerstoffkonzentration (1,43, 1,92 und 2,39 Vol%) bei konstanter Katalysatorbetteintrittstemperatur von 232°C erhalten wurden. Die übrigen Reaktionsbedingungen umfassten ein Verhältnis von Kohlenmonoxid zu Sauerstoff von 1,2 eine stündliche Gas-Raumgeschwindigkeit von 6.000 h⁻¹ und eine Länge eines Katalysatorbetts von 15 cm.

Während die zuvor zitierten Dokumente geeignete Katalysatoren für eine Sauerstoff- und Ethinentfernung und die Position einer solchen Entfernung in einem Gesamtprozess darlegen, stellt sich die vorliegende Erfindung die Aufgabe der Bereitstellung eines optimierten Betriebs- und Regelungskonzeptes für eine Rohgasbehandlung 50 im Rahmen eines Gesamtverfahrens, insbesondere bezüglich der nachfolgend zusammengefassten Aspekte.

Eine vorteilhafte Lösung soll insbesondere eine schnelle und zuverlässige Anpassung auf Schwankungen des Sauerstoffgehalts im Austritt des Umsetzungsschritts 10 (kurz- und langfristig) ermöglichen. Ferner soll eine einfache Regelbarkeit und Entkoppelung des Betriebes bzw. der Justierung des Umsetzungsschritts 10 und der Rohgasbehandlung 50 möglich sein. Ein weiterer wichtiger Aspekt ist die Sicherstellung der spezifikationsgerechten Entfernung von Sauerstoff und Ethin im Gesamtverfahren 100 bei möglichst geringen Ethenverlusten von weniger als 2%, weniger als 1,5%, weniger als 1% und insbesondere weniger als 0,5% im Einmaldurchgang sowie nur minimaler Nebenproduktbildung (insbesondere betreffend höhere Kohlenwasserstoffe) von in Summe weniger als 1000 Gew.-ppm.

Ein wichtiger Aspekt ist eine zuverlässige Vermeidung von kritischen Sauerstoffkonzentrationen. Insbesondere durch eine Anreicherung im Zerlegungsteil des Gesamtverfahrens 100 (z.B. im Kopfstrom eines Demethanizers 80). Dadurch ist auch ein optimierter Betrieb der Aminwäsche zur CO2 Entfernung möglich (keine Oxidation des Waschmittels durch Sauerstoff und somit nur minimaler Ersatz von Amin nötig). Weiter soll eine notwendige Mindesttemperatur bzw. eines notwendigen Mindesttemperaturanstieges in der Rohgasbehandlung 50 möglich sein. Eine Begrenzung der maximalen Katalysatorbett- bzw. Austrittstemperatur und Begrenzung des max. adiabaten Temperaturanstiegs zur Begrenzung des Ethenverlusts bzw. der Nebenproduktbildung ist möglich (Austrittstemperatur und zugleich Maximaltemperatur zwischen 300 und 400 °C, insbesondere zwischen 330 und 370 °C).

Die hier vorgeschlagenen Lösungen umfassen, die Rohgasbehandlung 50 unter Verwendung eines ein- oder mehrstufigen, adiabaten Festbettreaktors durchzuführen. Insbesondere kann eine einstufige Ausführung vorgesehen sein. Im Falle einer mehrstufigen Ausführung kann eine Temperaturanpassung (Kühlung) vor jeder weiteren Stufe vorgesehen sein.

Die Positionierung der Rohgasbehandlung 50 innerhalb eines Gesamtverfahrens 100 kann grundsätzlich nach einer Essigsäureabtrennung 20 erfolgen, also ohne weitere Druckerhöhung. Insbesondere kann jedoch eine Positionierung der Rohgasbehandlung 50 bei einem gegenüber dem Umsetzungsschritt 10 erhöhten Druck (5 bis 50 bara, 15 bis 40 bara, 20 bis 35 bara, 25 bis 35 bara) erfolgen. Vorgeschlagene Ausgestaltungen greifen dabei vorteilhafterweise auf ohnehin vorhandene Elemente des Gesamtverfahrens 100 zurück, indem eine Positionierung der Rohgasbehandlung 50 bevorzugt nach der Verdichtung 40 oder einer der Verdichtung 40 zugehörigen Verdichterstufe erfolgt.

Ein geeigneter Teilstrom aus dem Gesamtverfahren 100 kann dabei als Rückführstrom vor die Verdichtung bzw. insbesondere vor eine Verdichterstufe stromauf der Rohgasbehandlung 50 genutzt werden. Auf diese Weise können hohe Druckdifferenzen bzw. zusätzlicher Aufwand für eine separate Verdichtung eines Rückführstromes vermieden werden.

Ist die Rohgasbehandlung 50 stromauf mindestens einer Verdichterstufe angeordnet, kann sinngemäß in vergleichbarer Weise diese mindestens eine Verdichterstufe zum Ausgleich der Druckdifferenz genutzt werden. Ebenso kann aber auch ein eigenständiger Kreislaufverdichter für den Rückführstrom vorgesehen werden. Grundsätzlich kommen unterschiedliche Rückführströme in Frage, beispielsweise ein stromab der Rohgasbehandlung 50 abgezweigter Stoffstrom bzw. ein Stoffstrom, der nach einem in der Rohgasbehandlung 50 verwendeten adiabaten Festbettreaktor abgezweigt wird. Weitere Beispiele, die in Kombination oder alternativ hierzu verwendet werden können, umfassen einen oder mehrere Stoffströme, der oder die stromab der Kohlendioxidentfernung, der Trocknung und Kühlung 70, der Demethanisierung 80, einer Deethanisierung in Form eines Teilstroms eines Ethanrecycles, oder stromab eines Ethan-/Ethen-Splitters (insbesondere die Sumpffraktion, die aus Ethan besteht bzw. reich an Ethan ist, oder aus einem Seitenabzug) bereitgestellt werden. Im Fall einer Demethanisierung 80 kann insbesondere das über Kopf abgetrennte Leichtgas verwendet werden, was den zusätzlichen Vorteil einer Einstellmöglichkeit des Verhältnisses von Kohlenmonoxid zu Sauerstoff aufweist.

Die Menge des Rückführstromes kann im Verhältnis zum Eintrittsstrom der Rohgasbehandlung 50 zwischen 0 und 10, insbesondere zwischen 0 und 5, zwischen 0 und 2,5 oder zwischen 0 und 1 betragen, definiert als Verhältnis der Masse des Recyclestroms zur Masse des Einsatzstroms. Hier vorgeschlagene Maßnahmen beruhen zudem auf der Erkenntnis, dass Ethin immer ausreichend entfernt wird, wenn genug Sauerstoff am Eintritt einer Rohgasbehandlung 50 vorhanden ist.

Um eine ausreichende bzw. vollständige Ethinentfernung zu erzielen, ist in der Rohgasbehandlung 50 ein Überschuss an Sauerstoff im Vergleich zu Ethin notwendig. Insbesonder entspricht der Volumenanteil an Sauerstoff dabei mindestens dem 3-Fachen, mindestens dem 5-Fachen, mindestens dem 10-Fachen, bevorzugt mindestens den 20-fachen und besonders bevorzugt mindestens den 25-fachen Volumenanteil des Ethins.

In der Rohgasbehandlung 50 nicht durch oxidative Umsetzung von Ethin verbrauchter Sauerstoff reagiert sodann weiter mit Kohlenmonoxid sowie ggf. anderen Kohlenwasserstoffen ab. Auf diese Weise kann Sauerstoff immer auf einen notwenigen Zielwert nach der Rohgasbehandlung 50 reduziert werden. Wesentliches Reaktionsprodukt dieser exothermen Reaktionen ist immer Kohlendioxid.

Das Kohlendioxid wir in einer nachgeschalteten Kohlendioxidentfernung 60 entfernt. Aus reaktionskinetischen Gründen reagiert der Sauerstoff zunächst bevorzugt mit dem im Prozessgas befindlichen Kohlenmonoxid, bevor ggf. auch Kohlenwasserstoffe angegriffen werden, was dann zu Wertproduktverlust (Ethenverlust) führt. Daher sollte ein Mindestverhältnis von Kohlenmonoxid zu Sauerstoff vorliegen bzw. eingestellt werden, um diesen Wertproduktverlust gering zu halten. Vorteilhafte molare reaktionsstöchiometrische Verhältnisse (siehe oben) von Kohlenmonoxid zu Sauerstoff liegen insbesondere bei mindestens 0,5, mindestens 0,7, mindestens 0,9 oder mindestens 1,1, beispielsweise mindestens 1,25. Vorteilhafte Verhältnisse von Kohlenmonoxid zu Sauerstoff liegen bei höchstens 3,2 betragen, insbesondere höchstens 3,0.

Zur Vermeidung einer Reaktion von Sauerstoff mit Ethen, die zu einem Verlust dieses Wertproduktes führt, existiert ein vorteilhafter Temperaturbereich der Rohgasbehandlung 50, wobei es insbesondere gilt, eine maximale Temperatur nicht zu überschreiten. Ein vorteilhafter Betrieb der Rohgasbehandlung 50 erfolgt bei Eintrittstemperaturen (vgl. Figur 3) oberhalb von 160 °C, bevorzugt oberhalb von 180 °C, bevorzugt oberhalb von 200 °C. und besonders bevorzugt oberhalb von 230 °C. (hierbei handelt es sich, wie oben erwähnt, insbesondere um die SOR-Werte, d.h. im Betrieb ist eine Erhöhung möglich). Dabei kommt es entsprechend Figur 2 zu einem adiabatischen Temperaturanstieg, der insbesondere so gewählt wird, dass der Temperaturanstieg 40 bis 150 K, 60 bis 140 K und insbesondere 70 bis 120 K beträgt. Die Eintrittstemperatur, der Temperaturanstieg sowie die Austrittstemperatur können als geeignete Regelgrößen sowohl für eine Sauerstoffdosierung als auch die Einstellung des Recyclestromes dienen, wobei insgesamt eine maximale Katalysatorbetttemperatur (entspricht für einen adiabaten Reaktor der Austrittstemperatur) maximal 370 °C, insbesondere maximal 360 °C und weiter insbesondere maximal 350 °C nicht überschreiten sollte, um einerseits den Ethenverlust und andererseits die Bildung von Nebenprodukten zu begrenzen.

Weitere prozessrelevante Bedingungen umfassen bzw. sind insbesondere die stündliche Gas-Raumgeschwindigkeit (engl. Gaseous Hourly Space Velocity, GHSV, als Gesamtgasvolumenstrom des Einsatzgases unter Normbedingungen von 0 °C, 1,013 bara pro Katalysatorvolumen in den fachüblichen Einheiten (Nm³/h)gas/m³Kat bzw. h⁻¹) sowie der Prozessdruck. Die stündliche Gas-Raumgeschwindigkeit liegt insbesondere zwischen 1.000 und 15.000 h⁻¹, weiter insbesondere zwischen 2.000 und 12.000 h⁻¹ und beispielsweise zwischen 4.000 und 10.000 h⁻¹. Der Druck kann zwischen 5 und 50 bara, insbesondere zwischen 15 und 40 bara, weiter insbesondere zwischen 20 und 35 bara, und beispielsweise zwischen 25 und 35 bara.

Eine hier vorgeschlagene Einspeisung von Sauerstoff kann an einer geeigneten Position des Gesamtverfahrens 100 erfolgen. Vorteilhaft kann eine Einspeisung unmittelbar vor dem Eintritt in die Rohgasbehandlung 50 sein. Diese kann vor oder nach der Vorrichtung zur Rückführung eines Recyclestromes erfolgen und ist dann unabhängig von der Regelung des Recyclestromes, der insbesondere auch ausgeschaltet werden kann. Eine Position stromab der Kondensatabscheidung 20, also stromauf der Verdichtung 40 oder einer der Verdichtung 40 zugehörigen Verdichterstufe, ist ebenfalls möglich. Diese ist vorteilhaft, falls Sauerstoff nur auf einem reduzierten Druckniveau (wie z.B. für den Umsetzungsschritt 10 benötigt) bereitgestellt werden kann, kann jedoch mit möglichen Nachteilen hinsichtlich von Foulingeffekten in den Verdichterstufen behaftet sein. Unter reduziertem Druckniveau wird hier ein Druckniveau unterhalb des Druckes, bei dem die Rohgasbehandlung 50 erfolgt, verstanden. Wie bereits erwähnt ist alternativ auch die Verwendung eines eigenen Kreislaufverdichters 45 möglich.

Betriebstechnisch kann eine Onlinemessung sowohl des Sauerstoffgehaltes (z.B. in Form einer elektrochemischen Sauerstoffmesssung, paramagnetischen Sauerstoffmessung oder mittels Ramanspektroskopie, insbesondere auch unter Verwendung eines so genannten Quantenkaskadenlasers) als auch des Ethingehaltes (z.B. mittels Gaschromatographie oder Infrarotspektroskopie, insbesondere FTIR-Spektroskopie) relativ leicht und zuverlässig umgesetzt und in die Prozesssteuerung integriert werden. Ergänzend kann eine Messung der Ethin- und/oder Kohlenmonoxidkonzentration erfolgen (z.B. mittels Gaschromatographie, IR/Ramanspektroskopie, Quantenkaskadenlaser). Diese Messungen (einzeln oder in Kombination) sowie ein aus den erhaltenen Messwerten gebildetes Verhältnis von Sauerstoff zu Ethin und/oder Kohlenmonoxid zu Sauerstoff) lassen sich vorteilhaft als Regelungsgröße für eine Sauerstoffeinspeisung nutzen. Ebenso kann insbesondere die Sauerstoffmessung zur Einstellung eines Recyclestromes dienen, der zwischen 0 und einem Maximalwert geregelt werden kann. Ferner kann das Verhältnis von Kohlenmonoxid zu Sauerstoff zur Regelung der Rückführung eines kohlenmonoxidreichen Teilstromes vom Kopf eines Demethanizers 80 dienen. Mögliche Positionen für diese Messungen befinden sich stromauf der Rohgasbehandlung 50, insbesondere stromauf der Rückführung eines Recyclestromes und/oder stromab der Rückführung eines Recylestromes. Hier werden also die effektiven Konzentrationen am Eintritt in einen Festbettreaktor der Rohgasbehandlung 50 gemessen, was aufgrund der direkten Korrelation besonders vorteilhaft ist.

Zur Überwachung und Sicherstellung eines einwandfreien Betriebes kann zusätzlich eine Messung insbesondere der O2-Konzentration d/s der Rohgasbehandlung 50 erfolgen. Bedarfsweise kann auch die hier ermittelte Sauerstoffkonzentration als weitere Größe für die Regelung der Kreislaufmenge herangezogen werden. Eine Messung der Ethinkonzentration an dieser Stelle ist ebenfalls optional möglich.

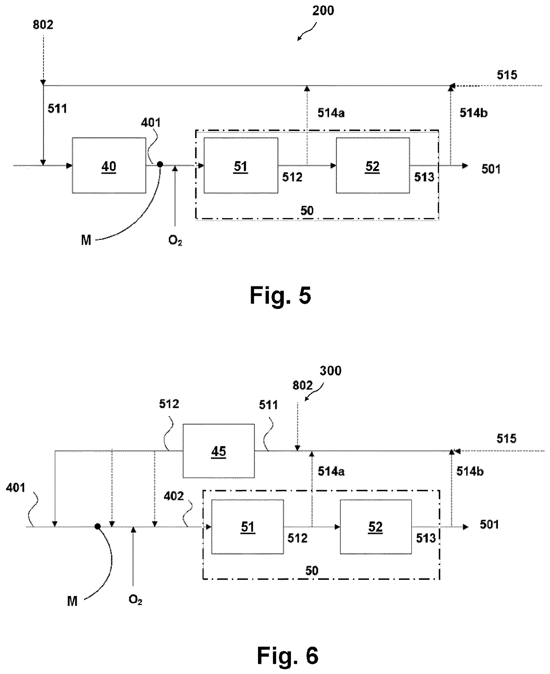

Hier vorgeschlagene Ausgestaltungen sind in den Figuren 5 und 6 veranschaulicht und mit 200 bzw. 300 bezeichnet. Die Einbindung in ein Gesamtverfahren 100, wie beispielsweise in Figur 1 veranschaulicht, ergibt sich dabei durch die gleiche Bezeichnung zumindest einiger der Stoffströme, wie beispielsweise 401 und 501, bzw. den gezeigten Verdichtungsschritt 40.

In der Ausgestaltung 200 gemäß Figur 5 ist die Rohgasbehandlung 50 nach einer Verdichtung 40 bzw. einer zugehörigen Verdichterstufe angeordnet und umfasst einen ersten Rohbehandlungsschritt 51 unter Verwendung eines entsprechenden Reaktors und optional einen zweiten Rohbehandlungsschritt 52. Im Beispiel der Figur 5 bzw. von Ausgestaltung 200 ist eine Rückführung vor die Verdichtung 40 oder eine beliebige, dieser zugehörigen Verdichterstufe dargestellt. Vor Eintritt in die Rohgasbehandlung 50 erfolgt im Beispiel, wie mit M bezeichnet, eine Messung des Sauerstoff-, Kohlenmonoxid- und/oder Ethingehalts im Strom 401.

Bei zu niedrigem Verhältnis von Sauerstoff zu Ethin oder bei zu geringem Sauerstoffgehalt für den optimalen Betrieb der Rohgasbehandlung 50 wird die Sauerstoffdosierung aktiviert bzw. erhöht. Diese Maßnahme wirkt im Gegensatz zu einer Temperaturanpassung unmittelbar und kann insbesondere Schwankungen im Sauerstoffgehalt des Eintrittsstromes 401 der Rohgasbehandlung 50, d.h. des dritten Komponentengemischs, (insbesondere resultierend aus Schwankungen in den vorgelagerten Strömen 102 und 201) ausgleichen. Bei zu hohen Sauerstoffgehalten kann hingegen die Sauerstoffdosierung reduziert bzw. auch abgeschaltet werden. In diesem Ausführungsbeispiel beinhaltet der Eintrittsstrom 401 bereits einen eventuellen Rückführstrom 511.

Ist die Sauerstoffkonzentration im Eintrittsstrom 401 der Rohgasbehandlung 50 weiterhin zu hoch und keine weitere Reduzierung der Sauerstoffeinspeisung mehr möglich (weil die Dosierung bereits deaktiviert ist und/oder bereits ein optimales Verhältnis von Sauerstoff zu Ethin erreicht ist), kann durch eine Aktivierung bzw. Erhöhung des Recylestromes 511 eine Reduzierung des Sauerstoffgehalts im Eintritt der Rohgasbehandlung 50 erfolgen. Dieser Recylestrom kann als Teilstrom 514a aus dem Austrittstrom 512 des ersten Rohbehandlungsschritts 51, als Teilstrom 514b aus dem Austrittsstrom 513 einer folgenden Stufe (hier beispielhaft dargestellt als zweiter Rohbehandlungsschritt 52) oder als Strom 515 wie oben erwähnt als Teilstrom aus einem der Rohgasbehandlung 50 nachgelagerten Verfahrensschritt gebildet werden. Insbesondere kann auch das Verhältnis von Kohlenmonoxid zu Sauerstoff durch gezielte Anpassung des kohlenmonoxidreichen Rückführstromes 802, der als Teilstrom des Kopfstromes 801 einer Demethanisierung 80 gebildet wird, eingestellt werden.

Erfolgt eine Rückführung eines Teilstromes 514a nach einem ersten Rohbehandlungsschritt 51, so kann ein folgender (optionaler) Rohbehandlungsschritt 52 insbesondere auch zur Feinreinigung dienen. Der Rückführstrom 511 kann ebenfalls aus einer geeigneten Kombination der Teilströme 514a, 514b, 515 und/oder 802 gebildet werden. In vergleichbarer Weise kann umgekehrt durch Reduzierung/Deaktivierung des Recylestromes 511 und Aktivierung /Erhöhung der O2-Einspeisung auf eine Verringerung des Sauerstoffgehaltes im Strom 401 reagiert werden bzw. das Verhältnis von Sauerstoff zu Ethin auf das für eine optimale Wirkung erforderliche minimale Verhältnis erhöht werden. In gleicher Weise kann das Verhältnis von Kohlenmonoxid zu Sauerstoff insbesondere durch Einstellung des Stromes 802 gezielt eingestellt werden. Notwendige Wärmetausche vor und nach der Rohgasbehandlung 50, bzw. in den Rückführströmen oder zwischen den Rohbehandlungsschritten 51 und 52 sind in dem Beispiel nicht dargestellt und können in dem Fachmann bekannter Weise ausgeführt werden.

Zu Figur 6 und Ausgestaltung 300 werden nur die Abweichungen gegenüber Figur 5 bzw. Ausgestaltung 200 erläutert. Falls ein separater Kreislaufverdichter 45 zum Einsatz kommt oder der Rohgasbehandlung 50 eine weitere Verdichterstufe (nicht dargestellt) folgt, kann die Rückführung eines Stroms 512 unmittelbar vor der Rohgasbehandlung 50 erfolgen, im Detail insbesondere vor oder nach einer Messung von Sauerstoff, Kohlenmonoxid und/oder Ethin sowie vor oder nach einer erfindungsgemäßen Vorrichtung zur Einspeisung von Sauerstoff. Der Strom 512 wird aus dem Strom 511 unter Erhöhung des Druckes gebildet.

In diesem Fall beinhaltet der Strom 401 keine Anteile aus einem Rückführstrom, sondern erst der Strom 402 wird aus der Kombination der Ströme 401 und 512 als unmittelbarer Einsatzstrom für die Rohgasbehandung 50 gebildet. Grundsätzlich ist auch die Verwendung mehrerer Kreislaufverdichter für unterschiedliche Teilströme 514a, 514b, 515 und/oder 802 denkbar, in der Praxis jedoch kaum relevant. Bevorzugt kommt also in dieser Ausführungsform nur ein Kreislaufverdichter 45 zum Einsatz bzw. übernimmt eine der Rohgasbehandlung 50 folgende Verdichterstufe 42 die Funktion des Kreislaufverdichters 45.

## Patentansprüche

1. Verfahren (100, 200, 300) zur oxidativen Dehydrierung von Ethan, wobei ein erstes Komponentengemisch unter Erhalt eines zweiten Komponentengemischs einem oder mehreren Umsetzungsschritten (10) unterworfen wird, wobei der eine oder die mehreren Umsetzungsschritte (10) die oxidative Dehydrierung umfasst oder umfassen, wobei das zweite Komponentengemisch oder ein Teil hiervon zur Bildung eines dritten Komponentengemischs verwendet wird, wobei das dritte Komponentengemisch einer Rohgasbehandlung (50) unterworfen wird, und wobei in der Rohgasbehandlung (50) Sauerstoff und Ethin in dem dritten Komponentengemisch umgesetzt werden, **dadurch gekennzeichnet, dass** die Rohgasbehandlung (50) unter Verwendung eines oder mehrerer Festbettreaktoren durchgeführt wird und dass ein Gehalt an Sauerstoff und Kohlenmonoxid in dem dritten Komponentengemisch eingestellt wird, indem ein oder mehrere rückgeführte vierte Komponentengemische und/oder Sauerstoff bei der Bildung des dritten Komponentengemischs verwendet wird oder werden.

2. Verfahren (100, 200, 300) nach Anspruch 1, wobei das erste Komponentengemisch Dampf, das Ethan und Sauerstoff umfasst, wobei das zweite Komponentengemisch einen Anteil des Ethans aus dem ersten Komponententemisch, der in dem einen oder den mehreren Umsetzungsschritten (10) nicht umgesetzt wurde, Ethen, Sauerstoff, Ethin, Essigsäure, Wasser, Kohlenmonoxid und Kohlendioxid umfasst, und wobei das dritte Komponentengemisch zumindest einen Teil des einen oder der mehreren Alkane und Alkene sowie des Sauerstoffs, Ethins und Kohlenmonoxids aus dem zweiten Komponentengemisch umfasst und gegenüber dem zweiten Komponentengemisch an Essigsäure und Wasser abgereichert ist.

3. Verfahren (100, 200, 300) nach einem der vorstehenden Ansprüche, wobei ein oder mehrere Teilströme eines oder mehrerer, in der Rohgasbehandlung (50) oder stromab hiervon gebildeter Stoffströme als das eine oder die mehreren vierten Komponentengemische verwendet wird oder werden.

4. Verfahren (100, 200, 300) nach einem der vorstehenden Ansprüche, wobei das eine oder die mehreren vierten Komponentengemische in einer Gesamtmenge rückgeführt wird oder werden, die weniger als das 10-Fache, 5-Fache, 3-Fache oder 1-Fache der Menge eines nicht durch das eine oder die mehreren vierten Komponentengemische gebildeten Anteils des dritten Komponentengemischs entspricht.

5. Verfahren (100, 200, 300) nach einem der vorstehenden Ansprüche, wobei das vierte Komponentengemische oder zumindest eines der mehreren vierten Komponentengemische gemeinsam mit oder separat von dem zweiten Komponentengemisch oder dem Teil hiervon, der zur Bildung des dritten Komponentengemischs verwendet wird, verdichtet wird oder werden.

6. Verfahren (100, 200, 300) nach einem der vorstehenden Ansprüche, wobei das vierte Komponentengemisch oder zumindest eines der mehreren vierten Komponentengemische unter Verwendung einer Leichtgasfraktion, die unter Verwendung eines stromab der Rohgasbehandlung (50) durchgeführten Demethanisierungsschritts (80) bereitgestellt wird, unter Verwendung eines stromab der Rohgasbehandlung (50) oder stromab eines der Rohgasbehandlung (50) zugehörigen adiabaten Festbettreaktors abgezweigten Stoffstroms, unter Verwendung eines stromab einer Kohlendioxidentfernung abgezweigten Stoffstroms, unter Verwendung eines stromab eines Trockners abgezweigten Stoffstroms, unter Verwendung eines stromab eines Deethanizers abgezweigten Stoffstroms, und/oder eines Ethanteilstroms eines Ethanrecycles eines C2-Splitters oder aus einem Seitenabzug eines C2-Splitters gebildet wird.

7. Verfahren (100, 200, 300) nach einem der vorstehenden Ansprüche, wobei bei der bei der Bildung des dritten Komponentengemischs verwendete Sauerstoff bei der Bildung des dritten Komponentengemischs einstellbar zudosiert wird.

8. Verfahren (100, 200, 300) nach einem der vorstehenden Ansprüche, wobei eine oder mehrere Mengen des einen oder der mehreren, bei der Bildung des dritten Komponentengemischs verwendeten vierten Komponentengemischs und/oder des bei der Bildung des dritten Komponentengemischs verwendeten Sauerstoffs auf Grundlage einer Messung einer Konzentration von Sauerstoff, Kohlenmonoxid und/oder Ethin stromauf der Rohgasbehandlung (50) und/oder auf Grundlage einer Messung eines adiabaten Temperaturanstieges in der Rohgasbehandlung (50) eingestellt wird oder werden.

9. Verfahren (100, 200, 300) nach einem der vorstehenden Ansprüche, wobei das vierte Komponentengemische oder zumindest eines der mehreren vierten Komponentengemische, das dritte Komponentengemisch, und/oder zumindest ein weiteres Komponentengemisch stromab der Rohgasbehandlung (50) unter Verwendung eines oder mehrerer Wärmetauscher temperiert wird oder werden.

10. Verfahren (100, 200, 300) nach einem der vorstehenden Ansprüche, wobei der Gehalt an Sauerstoff und Kohlenmonoxid in dem dritten Komponentengemisch derart eingestellt wird, dass ein Volumenanteil von Sauerstoff mindestens dem 3-Fachen eines Volumenanteils von Ethinentspricht und/oder ein molares reaktionsstöchiometrisches Verhältnis von Kohlenmonoxid zu Sauerstoff bei mindestens 0,5 liegt und/oder das dritte Komponentengemisch mit einer Mindesttemperatur von 160 °C der Rohgasbehandlung (50) zugeführt wird und/oder die Rohgasbehandlung (50) bei einer Maximaltemperatur von 370 °C betrieben wird und/oder bei dem die Rohgasbehandlung (50) derart betrieben wird, dass ein Temperaturanstieg in der Rohgasbehandlung (50) um 40 bis 150 K erfolgt.

11. Verfahren (100, 200, 300) nach einem der vorstehenden Ansprüche, wobei die Rohgasbehandlung (50) auf einem Absolutdruck von 5 bis 50 bar und/oder mit einer stündlichen Gas-Raumgeschwindigkeit von 1.000 bis 15.000 h⁻¹ betrieben wird und/oder wobei das dritte Komponentengemisch 25 bis 80 Vol.-%, 30-70 Vol.-% oder 40-60 Vol.% Ethen enthält und/oder die Rohgasbehandlung (50) derart durchgeführt wird, dass ein maximaler Ethenverlust in der Rohgasbehandlung (50) weniger als 2%, weniger als 1,5%, weniger als 1% oder weniger als <0,5% beträgt und/oder das vierte Komponentengemisch weniger als 1.000 vol.-ppb Ethin und/oder weniger als 100 vol.-ppm Sauerstoff enthält.

12. Verfahren (100, 200, 300) nach einem der vorstehenden Ansprüche, wobei an einem Austritt der Rohgasbehandlung (50) eine Sauerstoff- und/oder Ethinmessung durchgeführt wird und auf dieser Grundlage eine Menge des einen oder zumindest eines der mehreren rückgeführten vierten Komponentengemische eingestellt wird.

13. Verfahren (100, 200, 300) nach einem der vorstehenden Ansprüche, wobei die Rohgasbehandlung (50) unter Verwendung eines Katalysators durchgeführt wird, der mindestens eines der Elemente Kupfer, Mangan oder Ruthenium enthält.

14. Verfahren (100, 200, 300) nach einem der vorhergehenden Ansprüche, bei dem bei der Bildung des dritten Einsatzgemischs eine Verdichtung (40) und/oder Kondensatabscheidung (20) durchgeführt wird und/oder bei dem ein der Rohgasbehandlung (50) entnommenes Gasgemisch einer Kohlendixidentfernung (60) und/oder Trocknung (70) unterworfen wird und/oder bei dem ein getrocknetes Gasgemisch einer Demethanisierung (80) und/oder einer Auf- und/oder Abtrennung (90) von Kohlenwasserstoffen unterworfen wird.

15. Anlage zur oxidativen Dehydrierung von Ethan, die dafür eingerichtet ist, ein erstes Komponentengemisch unter Erhalt eines zweiten Komponentengemischs einem oder mehreren Umsetzungsschritten (10) zu unterwerfen, wobei der eine oder die mehreren Umsetzungsschritte (10) die oxidative Dehydrierung von Ethan umfasst oder umfassen, das zweite Komponentengemisch oder einen Teil hiervon zur Bildung eines dritten Komponentengemischs zu verwenden, das dritte Komponentengemisch einer Rohgasbehandlung (50) zu unterwerfen, und in der Rohgasbehandlung (50) Sauerstoff und Ethin in dem dritten Komponentengemisch umzusetzen, **dadurch gekennzeichnet, dass** für die Rohgasbehandlung (50) ein oder mehrere Festbettreaktoren bereitgestellt sind und dass Mittel bereitgestellt sind, die dafür eingerichtet sind, einen Gehalt an Sauerstoff und Kohlenmonoxid im dritten Komponentengemisch einzustellen, indem ein oder oder mehrere rückgeführte vierte Komponentengemische und/oder Sauerstoff bei der Bildung des dritten Komponentengemischs verwendet wird oder werden.
